(19) Europäisches Patentamt / European Patent Office / Office européen des brevets

(11) **EP 1 755 449 B1**

(12) **EUROPEAN PATENT SPECIFICATION**

(45) Date of publication and mention
of the grant of the patent:
**15.04.2015 Bulletin 2015/16**

(21) Application number: **05751276.6**

(22) Date of filing: **13.06.2005**

(51) Int Cl.:
**A61B 5/06** *(2006.01)*     **A61B 1/04** *(2006.01)*

(86) International application number:
**PCT/JP2005/011179**

(87) International publication number:
**WO 2005/120345 (22.12.2005 Gazette 2005/51)**

(54) **POSITION DETECTION SYSTEM FOR A MEDICAL DEVICE AND MEDICAL-DEVICE GUIDANCE SYSTEM**

POSITIONSNACHWEISSYSTEM FÜR EIN MEDIZINPRODUKT UND FÜHRUNGSSYSTEM FÜR EIN MEDIZINPRODUKT

SYSTÈME DE DÉTECTION DE POSITION D"UN DISPOSITIF MÉDICAL ET SYSTÈME DE GUIDAGE DE DISPOSITIF MÉDICAL

(84) Designated Contracting States:
**DE FR GB**

(30) Priority: **14.06.2004   JP 2004175519**
**11.04.2005   JP 2005113082**

(43) Date of publication of application:
**28.02.2007   Bulletin 2007/09**

(73) Proprietor: **Olympus Corporation**
**Tokyo 151-0072 (JP)**

(72) Inventor: **UCHIYAMA, Akio**
**Shibuya-ku,**
**Tokyo 151-0072 (JP)**

(74) Representative: **von Hellfeld, Axel et al**
**Wuesthoff & Wuesthoff**
**Patent- und Rechtsanwälte**
**Schweigerstrasse 2**
**81541 München (DE)**

(56) References cited:
**WO-A-2004/014225      US-A- 5 592 939**
**US-A1- 2002 198 676      US-B1- 6 522 907**

**Description**

TECHNICAL FIELD

The present invention relates to a position detection system for a medical device and to a medical-device guidance system.

BACKGROUND ART

Among medical devices, capsule endoscopes are medical devices of the type that are swallowed to enter a body to be examined, such as a subject, and pass through a passage inside a body cavity and that can capture images at a target position inside the passage in the body cavity. The capsule endoscope is configured to include an imaging device that can capture images, such as a CCD (Charge Coupled Device), and performs image capturing at a target site in the passage of the body cavity.

However, unless the capsule endoscope is guided, it cannot reach the target site, and therefore, it is necessary to detect the position of the capsule endoscope in the passage of the body cavity in order to guide it.

Accordingly, techniques have been proposed for detecting the position of capsule endoscopes and the like when guided to locations where the position cannot be visually confirmed (such as in a passage of a body cavity).

Japanese Patent No. 3321235 discloses a position detection technology for a capsule endoscope; the technology is formed of a capsule endoscope in which a magnetic-field detection coil is installed, an externally provided receiving unit (magnetic-field source) that generates a magnetic field to be detected by the magnetic-field detection coil, and a detection apparatus that determines the relative positions of the magnetic-field detection coil and the magnetic-field source.

Japanese Unexamined Patent Application Publication Nos. 2001-179700 and 2002-187100 disclose a motion control system for a movable micro-machine, including a magnetic-field generating unit that generates a rotation magnetic field; a robot main body that receives the rotation magnetic field that the magnetic-field generating unit generates to obtain a thrust by rotation; a position detection unit that detects the position of the robot main body; and a magnetic-field orienting unit that changes the orientation of the rotation magnetic field produced by the magnetic-field generating unit so as to be oriented in the direction in which the robot main body should move to reach the target.

In the technology described above, the robot main body is guided while controlling the orientation of the robot main body (capsule endoscope).

Furthermore, in order to simplify detection of the position of the capsule endoscope, a method has been proposed in which many driving coils and magnetic sensors are disposed outside the operating region of the capsule endoscope.

Document WO 2004/014225 A concerns a system and a method for monitoring and stimulating gastro-intestinal motility.

Document US 5,592,939 concerns a system for navigating a catheter probe through a body cavity which includes a sensing coil fixed to a distal end of the probe.

DISCLOSURE OF INVENTION

Some or all of the above problems are overcome by a position detection system according to claim 1, and a medical-device guidance system according to claim 16.

The present invention provides a position detection system for a medical device, having a simple configuration, that can detect the position and orientation of a medical device without using any electrical power of the medical device and that can detect the position thereof regardless of the position and orientation of the medical device, and also provides a medical-device guidance system that can accurately guide a medical device based on the detected position of the medical device.

The present invention provides the following compositions.

A first aspect of the present invention is a position detection system for a medical device that is inserted into a body of a subject, comprising a magnetic induction coil installed in the medical device; at least one driving coil, disposed outside an operating region of the medical device, that generate induced magnetism in the magnetic induction coil; and at least one magnetic sensor, disposed outside the operating region of the medical device, that detect the induced magnetism generated by the magnetic induction coil, wherein, when the medical device is disposed at individual positions in the operating region of the medical device, the driving coil cause magnetism to act on the magnetic induction coil from three or more different directions, and among the three or more directions in which the magnetism acts, at least one of the directions is arranged to be a direction intersecting a plane formed by the other two directions.

According to the first aspect of the invention, the driving coils cause the magnetic fields (alternating magnetic fields) produced by the driving coils to act on the magnetic induction coil from three or more different directions. Accordingly, the driving coils can produce induced magnetism in the magnetic induction coil due to the alternating magnetic fields from at least one direction, regardless of the direction in which the magnetic induction coil is oriented. For example, if the orientations of lines of magnetic force of the alternating magnetic fields from two different directions have a positional relationship wherein they are substantially orthogonal to the central axis of the magnetic induction coil and induced magnetism therefore cannot be generated in the magnetic induction coil, the driving coils cause the AC magnetic field from the one remaining direction to act on the magnetic induction coil, which allows induced magnetism to be generated.

As a result, since it is possible to always generate induced magnetism in the magnetic induction coil regardless of the orientation of the medical device, it is possible to always detect the induced magnetism with the magnetic sensors.

Also, for example, by disposing a plurality of magnetic sensors outside the medical device and detecting, all at once, the induced magnetism that the magnetic induction coil generates using the plurality of sensors, it is possible to obtain a plurality of pieces of information related to the position of the medical device, even though there is only one magnetic induction coil. With such a configuration, the position detection system can reduce the number of magnetic induction coils provided in the medical device and it is possible to reduce the number of components in the medical device.

More preferably, the position detection system is preferably configured so as to detect the induced magnetism all at once with six or more magnetic sensors. By doing so, the position detection system can obtain six pieces of information related to the position and orientation of the medical device, such as the spatial position, the orientation, the rotation, and so on.

Also, since the magnetic induction coil generates induced magnetism with the driving coils, which are disposed outside the medical device, the magnetic induction coil does not use any electrical power of the medical device. Accordingly, the position detection system can detect the position and orientation of the medical device without using any electrical power of the medical device.

In the first aspect of the invention described above, in a preferable configuration, a plurality of magnetic sensors is provided, and the plurality of magnetic sensors are disposed facing the operating region of the medical device in a plurality of directions.

By doing so, induced magnetism with a detectable intensity is exerted on the magnetic sensor disposed in at least one direction of the magnetic sensors disposed in the plurality of directions, regardless of the position at which the medical device (the magnetic induction coil) is disposed.

The intensity of the induced magnetism exerted on the magnetic sensor is affected by the distance between the medical device and the magnetic sensor and by the distance between the medical device and the driving coil. Accordingly, even if the position at which the medical device is disposed is a position where the induced magnetism exerted on the magnetic sensor disposed in the one direction is weak, a magnetic sensor disposed in another direction is at a position where the induced magnetism exerted on the magnetic sensor is not weak.

As a result, it is possible for a magnetic sensor to always detect the induced magnetism, regardless of the position at which the medical device is disposed.

Also, since the position detection system obtains the same number of pieces of magnetic field information as the number of magnetic sensors disposed at different positions, it is possible to obtain position information and the like of the medical device according to the number of pieces of magnetic field information.

The information obtained concerning the medical device can include a total of six pieces of information, for example, the X, Y, and Z coordinates of the medical device, the rotation phases $\phi$ and $\theta$ about two axes that are orthogonal to each other and orthogonal to the central axis of the magnetic induction coil, and the intensity of the induced magnetism. Accordingly, if six or more pieces of magnetic information are obtained, the six pieces of positional information described above can be determined; that is, it is possible to determine the position and orientation of the medical device and the intensity of the induced magnetism.

In the first aspect of the invention described above, the driving coils preferably generate induced magnetism in the magnetic induction coil over a frequency range near a resonance frequency of a resonance circuit that includes the magnetic induction coil.

By doing so, the frequency of the AC magnetic field is set to the frequency (resonance frequency) that causes resonance in the resonance circuit described above, and the position detection system can generate induced magnetism with a large amplitude from the magnetic induction coil, compared to other frequencies. Because the amplitude of the induced magnetism is large, the magnetic sensors can easily sense the induced magnetism and can easily detect the position of the medical device.

Since the frequency of the AC magnetic field varies over a range of frequencies near the resonance frequency, it is possible to cause resonance in the resonance circuit even if, for example, the resonance frequency of the resonance circuit changes due to changes in the environmental conditions (for example, the temperature conditions) or if there is a deviation in the resonance frequency due to individual differences in the resonance circuits.

In the configuration described above, it is preferable to include a magnetic-sensor selection unit that selectively uses the output of sensors detected to have high intensity of the induced magnetism among the induced magnetisms detected by the plurality of magnetic sensors.

By doing so, the position detection system selectively uses the output from the magnetic sensor that detects a high intensity of the detected induced magnetism and can thus reduce the amount of information to be computationally processed, which allows the computational load to be reduced. At the same time, since the level of computational processing is reduced, it is possible to shorten the time required for computation.

In the first aspect of the invention described above, the driving coils and the magnetic sensors are preferably disposed at opposing positions on either side of the operating region of the medical device.

By doing so, since the driving coils and the magnetic sensors are disposed opposite each other on either side of the operating region described above, it is possible to position the driving coils and the magnetic sensors such that they do not interfere in terms of the construction.

A second aspect of the present invention is a medical-device position detection system that repeatedly detects the position and orientation of the medical device, which is disposed in a body of a subject, comprising a coil, disposed inside the medical device, that generates a magnetic field; a plurality of magnetic sensors that repeatedly detect the magnetic field generated by the coil; a position calculating device that determines, by repeated calculation, the position and orientation of the medical device based on outputs of the magnetic sensors; and a magnetic-sensor selection unit that selects a magnetic sensor to use from the plurality of magnetic sensors to determine the position and orientation of the medical device in the calculation in the position calculating device.

According to the second aspect of the present invention, the position calculating device calculates the position and the orientation of the medical device based on the output from the magnetic sensor selected by the magnetic-sensor selection unit, and therefore, it is possible to reduce the amount of computation in the above-mentioned calculation.

Furthermore, the magnetic-sensor selection unit selects the magnetic sensor whose output is produced by the magnetic field that the coil generates, which reduces the amount of computations in the above-mentioned calculation, while maintaining the detection accuracy of the position and orientation of the medical device.

In the second aspect of the invention described above, it is preferable to include an alternating magnetic field generating device, provided outside the body of the subject, that generates an alternating magnetic field; a storage unit that stores an output indicating a magnetic field intensity that the magnetic sensor receives; and a change detection unit that determines the amount of change in the output of the magnetic sensor by subtracting the output stored in the storage unit from the output of the magnetic sensor; wherein the coil is a magnetic induction coil that generates an induced magnetic field by receiving the magnetic field generated by the alternating magnetic field generating device; the output of a magnetic sensor, when the magnetic sensor received only the alternating magnetic field, is stored in the storage unit; and the change detection unit determines the induced magnetic field generated by the coil by subtracting the output stored in the storage unit from the output of a magnetic sensor, when the magnetic sensor received the alternating magnetic field, and the induced magnetic field.

By doing so, the change detection unit can easily determine the output of only the induced magnetic field from the output of the magnetic sensor receiving both the AC magnetic field and the induced magnetic field, by calculating the difference between the output of the magnetic sensor receiving both the AC magnetic field and the induced magnetic field and the output of the magnetic sensor receiving only the AC magnetic field, which is stored in the storage unit.

In the second aspect of the invention described above, in a preferable configuration, the position calculating device estimates the output of the plurality of magnetic sensors at the next calculation time based on the position and the orientation of the medical device determined by the calculation; and the magnetic-sensor selection unit sets the magnetic sensor to be used when determining the position and the orientation of the medical device at the next calculation time based on the estimated outputs of the plurality of magnetic sensors.

By doing so, the position calculating device estimates the outputs of the plurality of magnetic sensors at the next calculation time based on the results of the position and orientation of the medical device determined by the calculations up to this point, and the magnetic-sensor selection unit selects the magnetic sensors to be used at the next calculation time based on that estimation result.

Accordingly, the position detection system can reduce the number of magnetic sensors used in the measurement at the next computation time, the measurement process can be simplified, and the computation of the position and the orientation of the medical device can thus be made faster. Moreover, the burden in terms of hardware of the position detecting system can be lessened, and the costs can be reduced.

In the configuration described above, preferably, the magnetic-sensor selection unit sequentially selects a predetermined number of the magnetic sensors for which the estimated outputs of the plurality of magnetic sensors are large.

By doing so, the magnetic-sensor selection unit sequentially selects the magnetic sensors having high outputs, which allows use of magnetic sensors for which the ratio of the output of the signal produced by the coil-generated magnetic field with respect to the noise is high. Accordingly, the amount of computation can be reduced without deteriorating the detection accuracy of the position and orientation of the medical device.

The predetermined number is preferably from six to ten, inclusive. By setting the number of magnetic sensors to at least six, it is possible to determine the position and orientation of a medical device having six degrees of freedom.

Furthermore, by setting the number of magnetic sensors to be more than six, it is possible to remove the effects of noise included in the outputs of the magnetic sensors. Moreover, by setting the number of magnetic sensors to be ten or less, it is possible to prevent the amount of computation in the position detection device from becoming excessively large.

In the configuration described above, preferably, the magnetic-sensor selection unit selects the magnetic sensors for which the estimated outputs of the plurality of magnetic sensors are larger than a predetermined value.

By doing so, the magnetic-sensor selection unit can select magnetic sensors having little noise, by selecting magnetic sensors whose outputs are larger than a predetermined value, and it is thus possible to select reliable magnetic sensors with high probability. Accordingly, the position detection system can improve the detection accuracy of the position and orientation of the medical device.

In the second aspect of the invention described above, in a preferable configuration, the position calculating device estimates the magnitude and the orientation of the magnetic field that the coil forms at the positions of individual magnetic sensors based on the position and the orientation determined by the calculation; and the magnetic-sensor selection unit selects the magnetic sensor to be used in determining the position and the orientation of the medical device at the next calculation time based on the estimated outputs of the individual magnetic sensors.

By doing so, the position calculating device estimates the magnitude and orientation of the magnetic field produced at the positions of the magnetic sensors at the next calculation time based on the results of the position and orientation of the medical device determined by the calculations up to this point, and the magnetic-sensor selection unit selects the magnetic sensors to be used at the next calculation time based on the result of that estimation.

Accordingly, the position detection system can reduce the number of magnetic sensors to be used in the measurement at the next calculation time and can simplify the measurement process, which allows the calculation of the position and orientation of the medical device to be made faster. Since the position detection system estimates the magnitude and the orientation of the magnetic fields produced at the positions of the magnetic sensors, the calculation process can be simplified compared to the method in which the output of each magnetic sensor is estimated, and the amount of computation in the position calculating device can thus be reduced.

In the configuration described above, preferably, the magnetic-sensor selection unit sequentially selects a predetermined number of the magnetic sensors for which the intensities of the magnetic fields that the coil forms at the positions of the plurality of magnetic sensors are large.

By doing so, the magnetic-sensor selection unit sequentially selects magnetic sensors in which the magnetic field intensity produced by the coil is large, which enables use of magnetic sensors for which the ratio of the output of the signal produced by the coil-generated magnetic field with respect to the noise is high. Accordingly, the position detection system can reduce the amount of computation without deteriorating the detection accuracy of the position and orientation of the medical device.

Since the position detection system estimates the magnitude and orientation of the magnetic field produced at the position of each magnetic sensor, the calculation process can be simplified compared to the method in which the output of each magnetic sensor is estimated, which allows the amount of computation in the position calculating device to be reduced.

In the second aspect of the invention described above, in a preferable configuration, the position calculating device estimates the distance between each magnetic sensor and the coil based on the position and the orientation of the medical device determined by the calculation; and the magnetic-sensor selection unit selects the magnetic sensor to be used in determining the position and the orientation of the medical device at the next calculation time based on the estimated distance between each magnetic sensor and the coil.

By doing so, by selecting magnetic sensors based on the distance between the magnetic sensors and the coil, the magnetic-sensor selection unit can use magnetic sensors for which the ratio of the output of the signal produced by the coil-generated magnetic field to the noise is high. Accordingly, the position detection system can reduce the amount of computation without deteriorating the detection accuracy of the position and orientation of the medical device.

Since the position detection system estimates the distance between each magnetic sensor and the coil, the calculation process can be simplified compared to the method in which the output of each magnetic sensor is estimated, which allows the amount of computation in the position calculating device to be reduced.

In the configuration described above, preferably, the magnetic-sensor selection unit selects the magnetic sensor for which the estimated distance between the magnetic sensor and the coil is smaller than a predetermined value.

By doing so, the magnetic-sensor selection unit selects magnetic sensors for which the distance between the magnetic sensor and the coil is shorter than a predetermined value, and therefore enables use of magnetic sensors for which the ratio of the output of the signal produced by the coil-generated magnetic field with respect to the noise is high. Accordingly, the position detection system can reduce the amount of computation without deteriorating the detection accuracy of the position and orientation of the medical device.

In the first aspect of the invention described above, preferably, the medical device is a capsule endoscope, and the longitudinal axis of the capsule endoscope and the direction of a central axis of the coil are made substantially the same.

By doing so, since the medical device is a capsule endoscope, it can observe the interior of the subject's body using the capsule endoscope.

For example, in the case of a substantially cylindrical capsule endoscope, the longitudinal axis direction of the capsule endoscope is the insertion direction.

A third aspect of the present invention is a medical-device guidance system comprising a position detection system according to the first aspect of the present invention; a magnet installed in the medical device; a magnetic-field generating unit, disposed outside the operating region of the medical device, that generates a magnetic field that is made to act on the magnet; and a magnetic-field-orientation control unit that controls the orientation of the magnetic field applied to the magnet by the magnetic-field generating unit.

By doing so, by controlling the orientation of the magnetic field acting on the magnet installed in the medical device, the medical-device guidance system can control the force acting on the magnet and can control the direction of motion of the medical device.

Since the medical-device guidance system can detect the position of the medical device at the same time, it can guide the medical device to a predetermined position.

In the third aspect of the invention described above, preferably, the magnetic-field generating unit includes three pairs of opposed frame-shaped electromagnets that are disposed in mutually orthogonal directions; a space in which the body of the subject can be positioned is provided inside the electromagnets; and the driving coils and the magnetic sensors are disposed in the periphery of the space in which the body of the subject can be positioned.

By doing so, the medical-device guidance system can control the orientation of the parallel magnetic fields generated inside the electromagnets in a predetermined direction by controlling the respective intensities of the magnetic fields generated from the three pairs of frame-shaped electromagnets disposed to oppose each other in mutually orthogonal directions. Accordingly, the medical-device guidance system can apply a magnetic field in a predetermined direction to the medical device, which allows the medical device to be moved in the predetermined direction.

Since the inside space between the electromagnets is a space in which the subject's body can be positioned, and the driving coils and the magnetic sensors are disposed in the periphery of this space, the medical-device guidance system can guide the medical device to a predetermined position inside the subject's body.

In the third aspect of the invention described above, a possible configuration is one in which the magnetic-field generating unit generates a rotation magnetic field around the medical device; and a spiral that converts rotary force about a longitudinal axis of the medical device into a propulsion force in the direction of the longitudinal axis is provided on the outer surface of the medical device.

By doing so, the medical-device guidance system can apply a rotation force to the medical device by generating a rotation magnetic field around the medical device by means of the magnetic-field generating unit. When a rotation force about the longitudinal axis is applied to the medical device, a force that propels the medical device in the direction of the longitudinal axis thereof is generated by operating the helical mechanism. Since the helical mechanism generates the propulsion force, by controlling the direction of rotation about the longitudinal axis, the direction of the propulsion force acting on the medical device can be controlled.

In the configuration described above, the medical device may include an imaging unit having an optical axis parallel to the longitudinal axis of the medical device; a display unit that displays an image captured by the imaging unit may be provided; and there may also be provided an image control unit that rotates the image captured by the imaging unit in an opposite direction and displays the image on the display unit, based on rotation information about the longitudinal axis of the medical device by the magnetic-field-orientation control unit.

By doing so, the medical-device guidance system performs processing to rotate the captured image described above in the rotation direction of the medical device or the reverse direction based on the rotation information (rotation phase information about the longitudinal axis). Accordingly, the medical-device guidance system can always display on the display unit an image acquired at a predetermined rotational phase, regardless of the rotational phase of the medical device.

For example, when guiding the medical device while the operator observes the image displayed on the display unit, converting the displayed image to an image with a predetermined rotational phase makes it easier to guide the medical device to a predetermined position compared to the case where the displayed image is an image that rotates along with the rotation of the medical device, as described above.

In the third aspect of the invention described above, preferably, the medical device is a capsule endoscope, and the longitudinal axis of the capsule endoscope and the direction of a central axis of the coil are made substantially the same.

By doing so, since the medical device is a capsule endoscope, it is possible to observe the interior of the subject's body using the capsule endoscope.

For example, if the capsule endoscope is substantially cylindrical, the direction of the longitudinal axis of the capsule endoscope is the insertion direction.

In the first aspect of the invention described above, it is preferable to include a driving-coil selecting unit that selectively uses a plurality of driving coils.

By doing so, the driving-coil selection unit can reduce the load and time required for computational processing by excluding the driving coil for which the orientation of the lines of magnetic force generated by the driving coil and the orientation of the central axis of the magnetic induction coil are substantially perpendicular, and by performing selection control that generates an AC magnetic field.

In the first aspect of the invention described above, preferably, a plurality of driving coils are disposed so as to be mutually orthogonal.

By doing so, the position detection system can simplify the processing and algorithm for the driving-coil selection control by the driving-coil selection unit.

In the third aspect of the invention described above, preferably, the driving coils and the magnetic sensors are disposed at the upper part of a space in which the subject can be positioned.

By doing so, since the subject is positioned at the lower part of the space in which the examinee can be disposed, it is possible to prevent the driving coils or the magnetic sensors, disposed at the upper part of the space in which the examinee can be disposed, from interfering with the subject.

BRIEF DESCRIPTION OF DRAWINGS

Fig. 1 is a schematic diagram of a capsule endoscope guidance system according to a first embodiment of the present invention.

Fig. 2 is a perspective view of a capsule endoscope guidance system.

Fig. 3 is a schematic diagram showing a cross-section of the capsule endoscope guidance system.

Fig. 4 is a schematic diagram showing the circuit layout of a magnetic-sensor receiving circuit.

Fig. 5 is a schematic diagram showing the configuration of a capsule endoscope.

Fig. 6 is a graph showing the frequency characteristic of a resonance circuit.

Fig. 7 is a diagram showing the positional relationship between a drive coil and a magnetic induction coil.

Fig. 8 is a diagram showing the positional relationship between drive coils and sense coils.

Fig. 9 is a diagram showing another positional relationship between the drive coils and the sense coils.

Fig. 10 is a diagram showing another positional relationship between the drive coils and the sense coils.

Fig. 11 is a cross-sectional view showing the configuration of a capsule endoscope.

Fig. 12 is a partial perspective view showing the configuration of an induced-magnetic-field generator.

Fig. 13 is a cross-sectional diagram showing another example of the configuration of a capsule endoscope.

Fig. 14 is a diagram showing the positional relationship between drive coils and sense coils according to a second embodiment of the present invention.

Fig. 15 is a schematic diagram showing the cross-section of a capsule endoscope guidance system.

Fig. 16 is a diagram showing the positional relationship between drive coils and sense coils according to a third embodiment of the present invention.

Fig. 17 is a diagram showing the positional relationship between drive coils and sense coils according to a modification of the third embodiment of the present invention.

Fig. 18 is a diagram schematically showing a position detection system of a capsule endoscope according to the present invention.

BEST MODE FOR CARRYING OUT THE INVENTION

First Embodiment

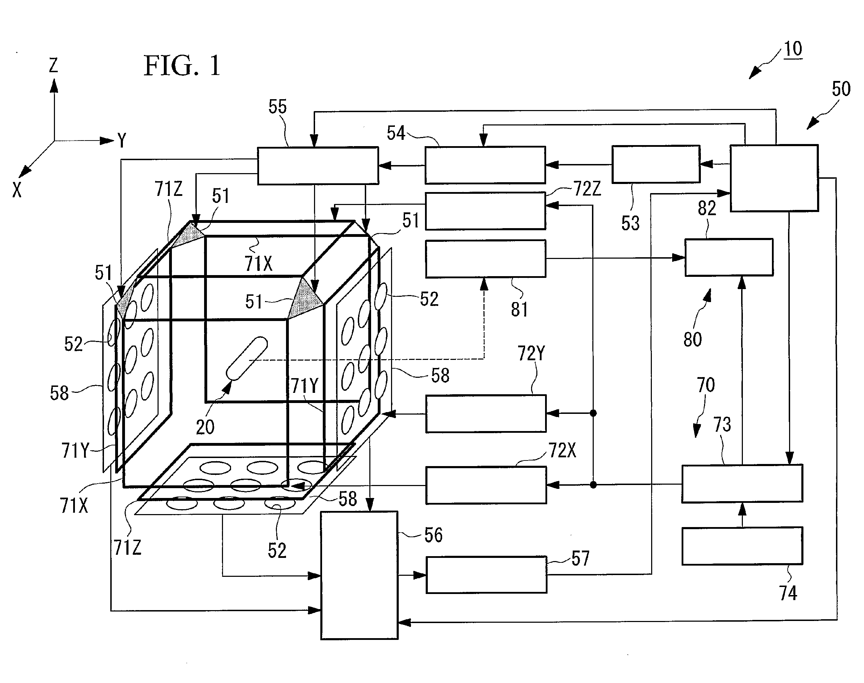

A first embodiment of a capsule endoscope guidance system according to the present invention will be described below with reference to Figs. 1 to 13.

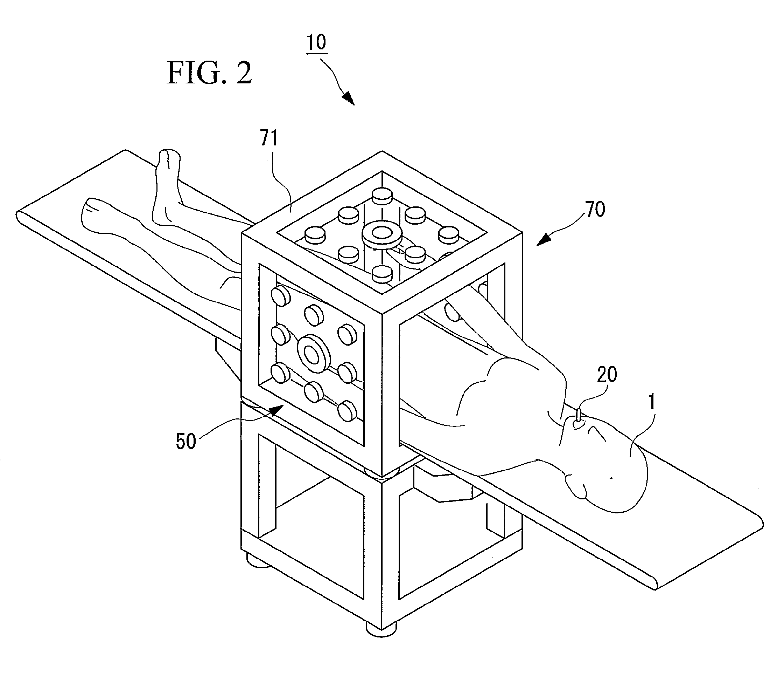

Fig. 1 is a diagram schematically showing a capsule endoscope guidance system according to this embodiment. Fig. 2 is a perspective view of the capsule endoscope guidance system.

As shown in Figs. 1 and 2, a capsule endoscope guidance system (medical-device guidance system) 10 is mainly formed of a capsule endoscope (medical device) 20 that is introduced into a body cavity of a subject (specimen) 1, per oral or per anus, to optically image an internal surface of a passage in the body cavity and wirelessly transmit an image signal; a position detection apparatus (position calculating apparatus, change detector) 50 that detects the position of the capsule endoscope 20; a magnetic induction apparatus 70 that guides the capsule endoscope 20 based on the detected position of the capsule endoscope 20 and instructions from an operator; and an image display device 80 that displays the image signal transmitted from the capsule endoscope 20.

As shown in Fig. 1, the magnetic induction apparatus 70 is mainly formed of a three-axis Helmholtz coil unit (magnetic-field generator, electromagnet) 71 that produce parallel magnetic fields for driving the capsule endoscope 20; a Helmholtz-coil driver 72 that controls the gain of currents supplied to the three-axis Helmholtz coil unit 71; a rotation-magnetic-field control circuit (magnetic-field-orientation control unit) 73 that controls the directions of the parallel magnetic fields for driving the capsule endoscope 20; and an input device 74 that outputs to the rotation-magnetic-field control circuit 73 the direction of movement of the capsule endoscope 20 that the operator inputs.

As shown in Figs. 1 and 2, the three-axis Helmholtz coil unit 71 is formed in a substantially rectangular shape. The three-axis Helmholtz coil unit 71 includes three-pairs of mutually opposing Helmholtz coils 71X, 71Y, and 71Z, and each pair of Helmholtz coils 71X, 71Y, and 71Z is disposed so as to be substantially orthogonal to the X, Y, and Z axes in Fig. 1. The Helmholtz coils disposed substantially orthogonally with respect to the X, Y, and Z axes are denoted as the Helmholtz coils 71X, 71Y, and 71Z, respectively.

The Helmholtz coils 71X, 71Y, and 71Z are disposed so as to form a rectangular space in the interior thereof. As shown in Fig. 1, the rectangular space serves as an operating space of the capsule endoscope 20, and as shown in Fig. 2, is the space in which the subject 1 is placed.

Although Helmholtz coils are indicated in this embodiment, as shown in Figs. 1 and 2, they may be formed of square coils, and it is not necessary that they strictly satisfy the conditions of Helmholtz coils.

The Helmholtz coil driver 72 includes Helmholtz coils drivers 72X, 72Y, and 72Z for controlling the Helmholtz coils 71X, 71Y, and 71Z, respectively.

Direction-of-movement instructions for the capsule endoscope 20, which the operator inputs from the input device 74, are input to the rotation-magnetic-field control circuit 73, together with data from the position detection apparatus 50 indicating the direction in which the capsule endoscope 20 is currently pointing (the direction of a rotation axis (longitudinal axis) R of the capsule endoscope 20). Then, signals for controlling the Helmholtz coil drivers 72X, 72Y, and 72Z are output from the rotation-magnetic-field control circuit 73, and rotational phase data of the capsule endoscope 20 is output to the image display device 80.

An input device for specifying the direction of movement of the capsule endoscope 20 by moving a joystick is used as the input device 74.

As mentioned above, the input device 74 may use a joystick-type device, or another type of input device may be used, such as an input device that specifies the direction of movement by pushing direction-of-movement buttons.

As shown in Fig. 1, the position detection apparatus 50 is mainly formed of drive coils (driving coils) 51 that generate induced magnetism in a magnetic induction coil (described later) in the capsule endoscope 20; sense coils (magnetic sensors) 52 that detect the induced magnetism generated in the magnetic induction coil; and the position detection apparatus 50 that computes the position of the capsule endoscope 20 based on the induced magnetism that the sense coils 52 detect and that controls the alternating magnetic field formed by the drive coils 51.

Between the position detection apparatus 50 and the drive coils 51 there are provided a sine-wave generating circuit 53 that generates an AC current based on the output from the position-detecting device 50; a drive-coil driver 54 that amplifies the AC current input from the sine-wave generating circuit 53 based on the output from the position detection apparatus 50; and a drive-coil selector 55 that supplied the AC current to a drive coil 51 selected on the basis of the output from the position detection apparatus 50.

Between the sense coils 52 and the position detection apparatus 50 there are provided a sense-coil selector (magnetic-sensor selecting unit) 56 that selects from the sense coils 52 AC current that includes position information of the capsule endoscope and so on, based on the output from the position detection apparatus 50; and a sense-coil receiving circuit 57 that extracts an amplitude value from the AC current passing through the sense-coil selector 56 and outputs it to the position detection apparatus 50.

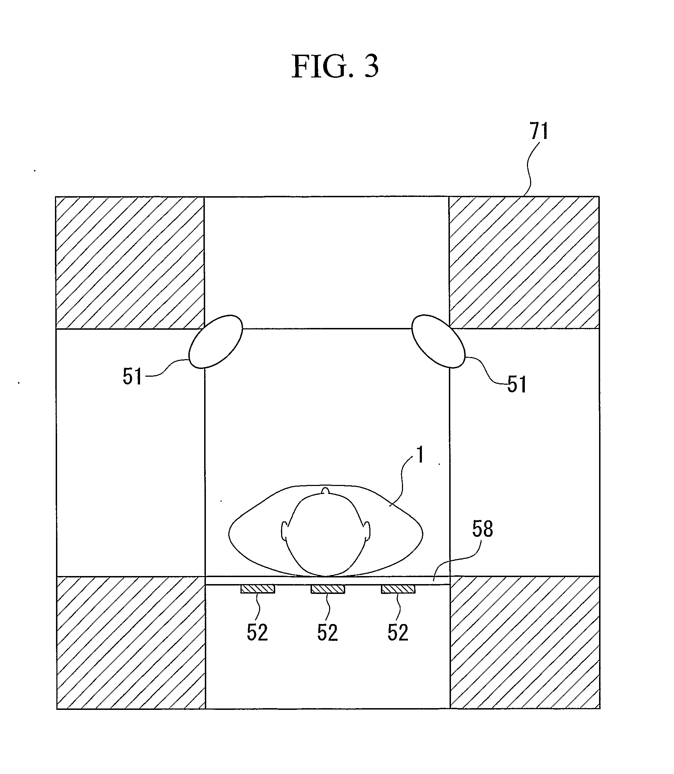

Fig. 3 is a schematic diagram showing a cross-section of the capsule endoscope guidance system.

Here, as shown in Figs. 1 to 3, the drive coils 51 are positioned at an angle at the four upper (in the normal direction of the Z-axis) corners of the substantially rectangular operating space formed by the Helmholtz coils 71X, 71Y, and 71Z. The drive coils 51 form substantially triangular coils that connect the corners of the square-shaped Helmholtz coils 71X, 71Y, and 71Z. By disposing the drive coils 51 at the top in this way, it is possible to prevent interference between the drive coils 51 and the subject 1.

The drive coils 51 may be substantially triangular coils, as mentioned above, or it is possible to use coils of various shapes, such as circular coils, etc.

The sense coils 52 are formed as air-core coils, and are supported, at the inner side of the Helmholtz coils 71X, 71Y, and 71Z, by three planar coil-supporting parts 58 that are disposed at positions facing the drive coils 51 and at positions mutually opposing each other in the Y-axis direction, with the operating space of the capsule endoscope 20 being disposed therebetween. Nine of the sense coils 52 are arranged in the form of a matrix in each coil-supporting part 58, and thus a total of 27 sense coils 52 are provided in the position detection apparatus 50.

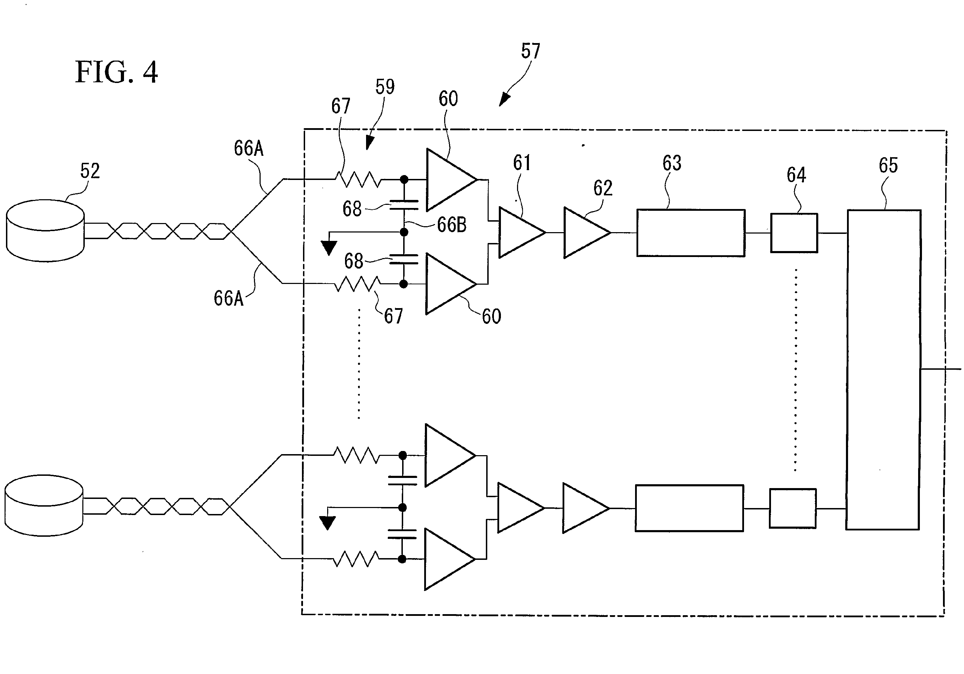

Fig. 4 is a schematic diagram showing the circuit configuration of the sense-coil receiving circuit 57.

As shown in Fig. 4, the sense-coil receiving circuit 57 is formed of a high-pass filter (HPF) 59 that removes low-frequency components of input AC voltages including the position information of the capsule endoscope 20; pre-amplifiers 60 that amplify the AC voltages; a band-pass filter (BPF) 61 that removes high-frequencies included in the amplified AC voltages; an amplifier (AMP) 62 that amplifies the AC voltage from which the high frequencies have been removed; a root-mean-square detection circuit (True RMS converter) 63 that detects the amplitude of the AC voltage and that extracts and outputs an amplitude value; an A/D converter 64 that converts the amplitude value to a digital signal; and a memory 65 for temporarily storing the digitized amplitude value.

The high-pass filter 59 is formed of resistors 67 disposed in a pair of wires 66A extending from the sensor coil 52; a wire 66B that is connected to the pair of wires 66A and that is grounded substantially at the center thereof; and a pair of capacitors 68 disposed opposite each other, with the connection point to the wire 66B therebetween. The pre-amplifiers 60 are disposed in the pair of wires 66A, respectively, and the AC voltages output from the pre-amplifiers 60 are input to the single band-pass filter 61. The memory 65 temporarily stores the amplitude values obtained from the nine sensor coils 52, and outputs the stored amplitude values to the position detection apparatus 50.

The root-mean-square detection circuit 63 may be used to extract the amplitude value of the AC voltage, as mentioned above, the amplitude value may be detected by smoothing the magnetic information using a rectifying circuit and detecting the voltage, or the amplitude value may be detected using a peak detecting circuit that detects a peak in the AC voltage.

Regarding the waveform of the detected AC voltage, the phase with respect to a waveform applied to the drive coil 51 changes depending on the presence of the magnetic induction coil 42 and the position. This phase change may be detected with a lock-in amplifier or the like.

As shown in Fig. 1, the image display device 80 is formed of an image receiving circuit 81 that receives the image transmitted from the capsule endoscope 20, and a display unit (image control unit) 82 that displays the image based on the received image signal and a signal from the rotation-magnetic-field control circuit 73.

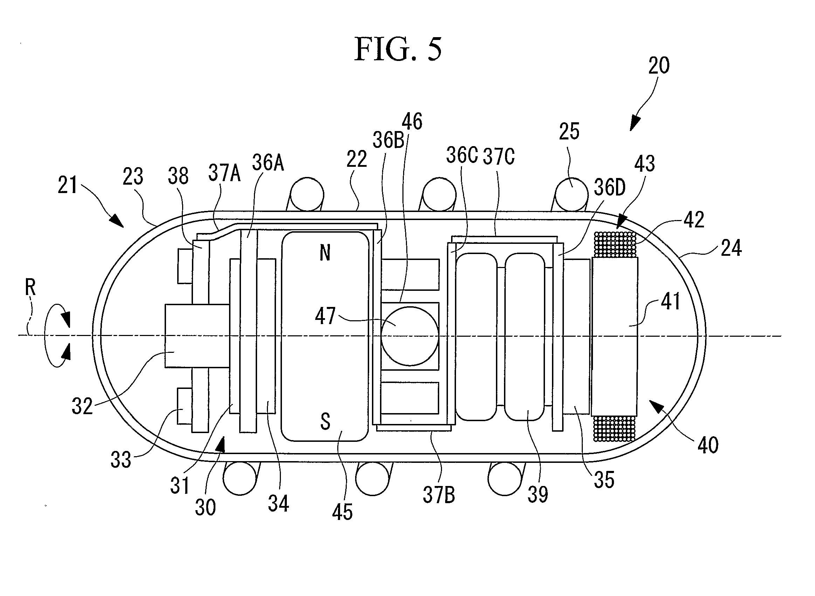

Fig. 5 is a schematic diagram showing the configuration of the capsule endoscope.

As shown in Fig. 5, the capsule endoscope 20 is mainly formed of an outer casing 21 that accommodates various devices in the interior thereof; an imaging unit 30 that images an internal surface of a passage in the body cavity of the subject; a battery 39 for driving the imaging unit 30; an induced-magnetism generator 40 that generates induced magnetism by means of the drive coils 51 described above; and a driving magnet (magnet) 45 that drives the capsule endoscope 20.

The outer casing 21 is formed of an infrared-transmitting cylindrical capsule main body (hereinafter abbreviated simply as main body) 22 whose central axis defines a rotation axis (longitudinal axis) R of the capsule endoscope 20, a transparent hemispherical front end portion 23 that covers the front end of the main body 22, and a hemispherical rear end portion 24 that covers the rear end of the main body, to form a sealed capsule container with a watertight construction.

A helical part (helical mechanism) 25 in which a wire having a circular cross-section is wound in the form of a helix about the rotation axis R is provided on the outer circumferential surface of the main body of the outer casing 21.

The imaging unit 30 is mainly formed of a board 36A positioned substantially orthogonal to the rotation axis R; an image sensor 31 disposed on the surface at the front end portion 23 side of the board 36A; a lens group 32 that forms an image of the internal surface of the passage inside the body cavity of the subject on the image sensor 31; an LED (Light Emitting Diode) 33 that illuminates the internal surface of the passage inside the body cavity; a signal processing unit 34 disposed on the surface at the rear end portion 24 side of the board 36A; and a radio device 35 that transmits the image signal to the image display device 80.

The signal processing unit 34 is electrically connected to the battery 39 via boards 36A, 36B, 36C, and 36D and flexible boards 37A, 37B, and 37C, is electrically connected to the image sensor 31 via the board 36A, and is electrically connected to the LED 33 via the board 36A, the flexible board 37A, and a support member 38. Also, the signal processing unit 34 compresses the image signal that the image sensor 31 acquires, temporarily stores it (memory), and transmits the compressed image signal to the exterior from the radio device 35, and in addition, it controls the on/off state of the image sensor 31 and the LED 33 based on signals from a switch unit 46 to be described later.

The image sensor 31 converts the image signal imaged via the front end portion 23 and the lens group 32 to an electrical signal (image signal) and outputs it to the signal processing unit 34. CMOS (Complementary Metal Oxide Semiconductor) or CCDs (Charge Coupled Devices), for example, can be used as this image sensor 31.

Moreover, a plurality of the LEDs 33 are disposed on the support member 38 positioned towards the front end portion 23 from the board 36A such that gaps are provided therebetween in the circumferential direction around the rotation axis R.

The driving magnet 45 is disposed at the rear end portion 24 side of the signal processing unit 34. The driving magnet 45 is disposed or polarized so as to have a magnetization direction in a direction orthogonal to the rotation axis R (for example, in the vertical direction in Fig. 5).

A switch unit 46, which is disposed on the board 36B, is provided at the rear end portion 24 side of the driving magnet 45. The switch unit 46 has an infrared sensor 47 and is electrically connected to the signal processing unit 34 via the board 36B and the flexible board 37A and is electrically connected to the battery 39 via the boards 36B, 36C, and 36D and the flexible boards 37B and 37C.

Also, a plurality of the switching units 46 are disposed in the circumferential direction about the rotation axis R at regular intervals, and the infrared sensor 47 is disposed so as to face the outside in the diameter direction. In this embodiment, an example has been described in which four switch units 46 are disposed, but the number of switch units 46 is not limited to four; any number may be provided.

At the rear end portion 24 side of the switch units 46, the battery 39 is disposed so as to be sandwiched by the boards 36C and 36D.

The radio device 35 is disposed on the surface of the board 36D at the rear end portion 24 side. The radio device 35 is electrically connected to the signal processing unit 34 via the boards 36A, 36B, 36C, and 36D and the flexible boards 37A, 37B, and 37C.

The induced-magnetism generator 40 is disposed at the rear end portion 24 side of the radio device 35. The induced-magnetism generator 40 is formed of a core member 41 made of ferrite formed in the shape of a cylinder whose central axis is substantially the same as the rotation axis R; an magnetic induction coil (coil) 42 that is disposed at the outer circumferential part of the core 41; and a capacitor (not shown in the drawing) that is electrically connected to the magnetic induction coil 42 and that forms the resonance circuit 43.

Next, the operation of the capsule endoscope guidance system 10 having the above-described configuration will be described.

First, an overview of the operation of the capsule endoscope guidance system 10 will be described.

As shown in Figs. 1 and 2, the capsule endoscope 20 is inserted, per oral or per anus, into a body cavity of a subject 1 who is lying down inside the position detection apparatus 50 and the magnetic induction apparatus 70. The position of the inserted capsule endoscope 20 is detected by the position detection apparatus 50, and it is guided to the vicinity of an affected area inside a passage in the body cavity of the subject 1 by the magnetic induction apparatus 70. The capsule endoscope 20 images the internal surface of the passage in the body cavity while being guided to the affected area and in the vicinity of the affected area. Then, data for the imaged internal surface of the passage inside the body cavity and data for the vicinity of the affected area are transmitted to the image display apparatus 80. The image display apparatus 80 displays the transmitted images on the display unit 82.

Next, the operation of the position detection apparatus 50 will be described.

As shown in Fig. 1, in the position detection apparatus 50, the sine-wave generator circuit 53 generates an alternating current based on the output from the position detection apparatus 50, and the alternating current is output to the drive-coil driver 54. The frequency of the generated alternating current is in a frequency range from a few kHz to 100 kHz, and the frequency varies (sweeps) within the above-mentioned range over time, so as to include a resonance frequency to be described later. The sweep range is not limited to the range mentioned above; it may be a narrower range or it may be a wider range, and is not particularly limited.

The alternating current is amplified in the drive-coil driver 54 based on an instruction from the position detection apparatus 50 and is output to the drive-coil selector 55. The amplified alternating current is supplied to the drive coil 51 selected by the position detection apparatus 50 in the drive-coil selector 55. Then, the alternating current supplied to the drive coil 51 produces an AC magnetic field in the operating space of the capsule endoscope 20.

An induced electromotive force is produced in the magnetic induction coil 42 of the capsule endoscope 20 disposed in the AC magnetic field due to the AC magnetic field, and an induced current flows therein. When the induced current flows in the magnetic induction coil 42, induced magnetism is produced by the induced current.

Since the magnetic induction coil 42 forms the resonance circuit 43 together with the capacitor, when the synchronization of the AC magnetic field matches the resonance frequency of the resonance circuit 43, the induced current flowing in the resonance circuit 43 (the magnetic induction coil 42) increases, and the induced magnetism produced also intensifies. Furthermore, since the core member 41, which is formed of dielectric ferrite, is disposed at the center of the magnetic induction coil 42, the induced magnetism is more easily concentrated in the core member 41, and the induced magnetism produced thus becomes even more intense.

The induced magnetism described above produces an induced electromotive force in the sense coil 52, and an AC voltage (magnetic information) that includes position information of the capsule endoscope 20 and so on is produced in the sense coil 52. This AC voltage is input to the sense-coil receiving circuit 57 via the sense-coil selector 56, where an amplitude value (amplitude information) of the AC voltage is extracted.

As shown in Fig. 4, low frequency components included in the AC voltage input to the sense-coil receiving circuit 57 are first removed by the high-pass filter 59, and the AC voltage is then amplified by the pre-amplifiers 60. Thereafter, high frequencies are removed by the band-pass filter 61, and the AC voltage is amplified by the amplifier 62. The amplitude value of the AC voltage from which unwanted components have been removed in this way is extracted by the root-mean-square detection circuit 63. The extracted amplitude value is converted to a digital signal by the A/D converter 64 and is stored in the memory 65.

The memory 65 stores, for example, an amplitude value corresponding to one period in which the sine-wave signal generated in the sine-wave generating circuit 53 is swept close to the resonance frequency of the resonance circuit 43, and outputs the amplitude value for one period at a time to the position detection apparatus 50.

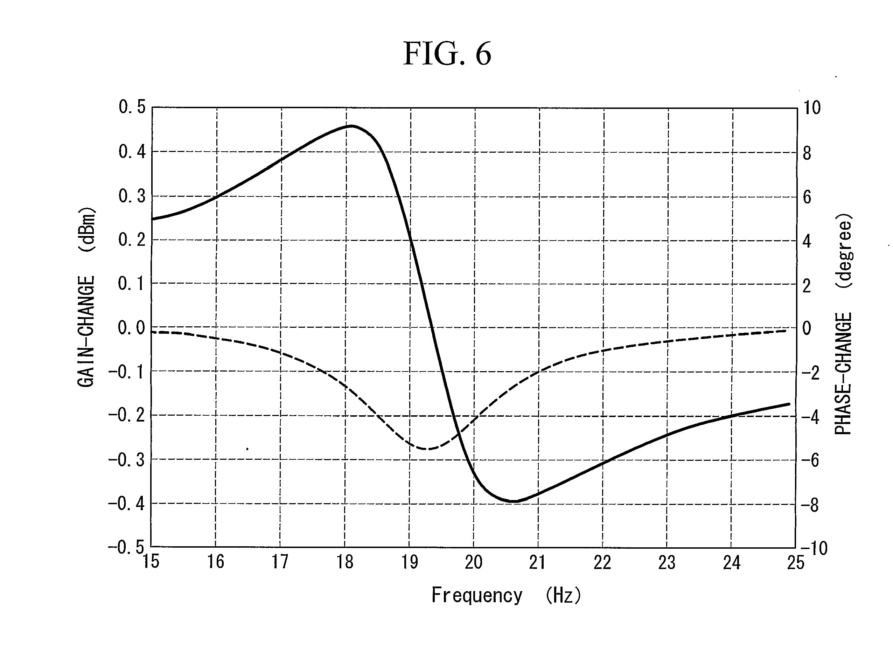

As shown in Fig. 6, the amplitude value of the AC voltage strongly varies depending on the relationship between the AC magnetic field that the drive coil 51 generates and the resonance frequency of the resonance circuit 43. Fig. 6 shows the frequency of the AC magnetic field on the horizontal axis and the variations in gain (dBm) and phase (degree) of the AC voltage flowing in the resonance circuit 43 on the vertical axes. It is shown that the variation in gain, indicated by the solid line, exhibits a maximum value at a frequency smaller than the resonance frequency, is zero at the resonance frequency, and exhibits a minimum value at a frequency higher than the resonance frequency. Also, it is shown that the variation in phase, indicated by the broken line, drops most at the resonance frequency.

Depending on the measurement conditions, there may be cases where the gain exhibits a minimum value at a frequency lower than the resonance frequency and exhibits a maximum value at a frequency higher than the resonance frequency, and where the phase reaches a peak at the resonance frequency.

The extracted amplitude value is output to the position detection apparatus 50, and the position detection apparatus 50 assumes the amplitude difference between the maximum value and the minimum value of the amplitude value in the vicinity of the resonance frequency as the output from the sense coil 52. Then, the position detection apparatus 50 obtains the position and so forth of the capsule endoscope 20 by solving simultaneous equations involving the position, direction, and magnetic field strength of the capsule endoscope 20 based on the amplitude difference obtained from the plurality of sense coils 52.

Thus, by setting the output of the sense coils 52 as the amplitude difference in this way, it is possible to cancel variations in amplitude that originate from variations in the magnetic field intensity due to environmental conditions (for example, temperature), and it is therefore possible to obtain the position of the capsule endoscope 20 with a stable degree of accuracy without being affected by environmental conditions.

The information on the position and so forth of the capsule endoscope 20 includes six pieces of information, for example, X, Y, and Z positional coordinates, rotational phases φ and θ about axes that are orthogonal to each other and orthogonal to the longitudinal axis (rotation axis) of the capsule endoscope 20, and the intensity of the induced magnetism that the magnetic induction coil 42 produces.

In order to estimate these six pieces of information by calculation, the outputs of at least six sense coils 52 are necessary. Since the outputs of nine sense coils 52 disposed in at least one plane are used to estimate the position of the capsule endoscope 20, it is possible to obtain the six pieces of information mentioned above by calculation.

The position detection apparatus 50 instructs the amplification factor of the alternating current supplied to the drive coil 51 to the drive-coil driver 54 based on the position of the capsule endoscope 20 obtained by calculation. This amplification factor is set so that the induced magnetism produced by the magnetic induction coil 42 can be detected by the sense coil 52.

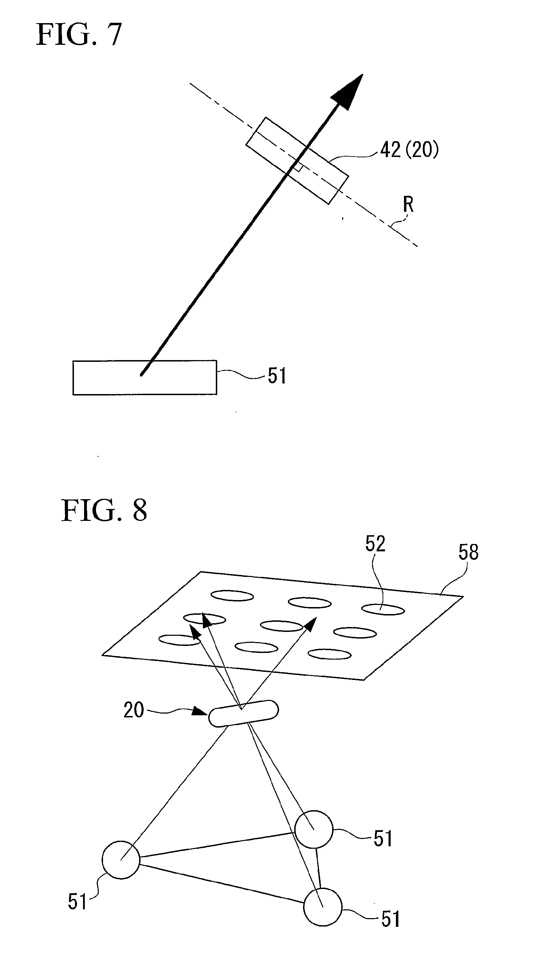

Also, the position detection apparatus 50 selects the drive coil 51 to produce the magnetic field, and outputs to the drive coil selector 55 an instruction for supplying the alternating current to the selected drive coil 51. As shown in Fig. 7, in the method of selecting the drive coil 51, a drive coil 51 for which a straight line (orientation of the drive coil 51) connecting the drive coil 51 and the magnetic induction coil 42 and the central axis of the magnetic induction coil 42 (the rotation axis R of the capsule endoscope 20) are substantially orthogonal is excluded. In addition, as shown in Fig. 8, the drive coils 51 are selected so as to supply the alternating current to three of the drive coils in such a way that the orientations of the magnetic fields acting on the magnetic induction coil 42 are linearly independent.

A more preferable method is a method in which a drive coil 51 for which the orientation of the lines of magnetic force produced by the drive coils 51 and the central axis of the magnetic induction coil 42 are substantially orthogonal is omitted.

The number of drive coils 51 forming the AC magnetic field may be limited using the drive-coil selector 55, as described above, or the number of drive coils 51 disposed may be initially set to three without using the drive-coil selector 55.

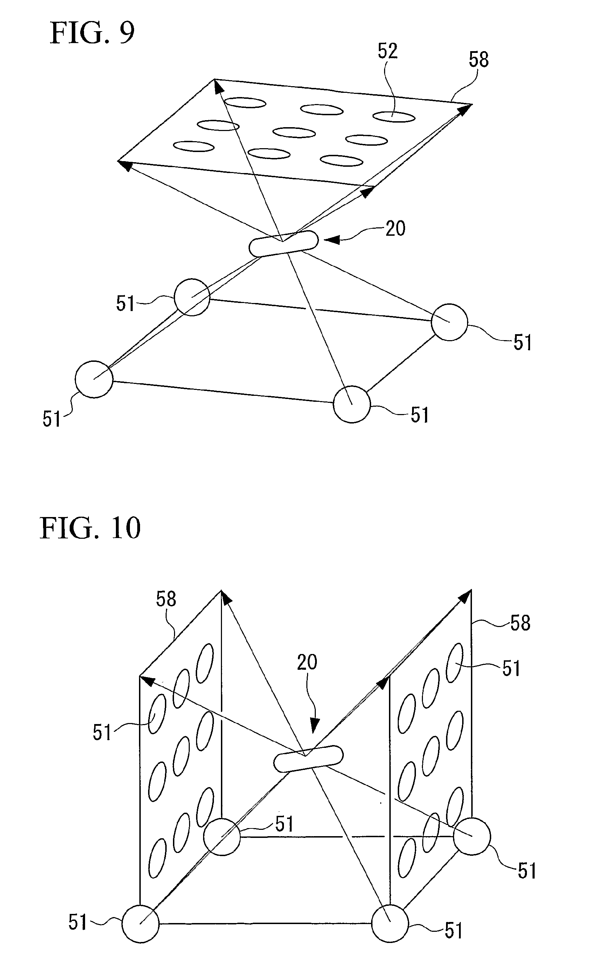

As described above, three drive coils 51 may be selected to form the AC magnetic field, or as shown in Fig. 9, the AC magnetic field may be produced by all of the drive coils 51.

Next, the method of selecting the sense coil 52 used in estimation of the position and orientation of the capsule endoscope 20 by means of the position detection apparatus will be described.

Here, in order to simplify the description, identifiers are assigned to the sense coils 52. Since there are 27 sense coils 52, as described above, identifiers from s1 to s27 are assigned to the sense coils 52.

Regarding the position and orientation in the space between the sense coils 52, for the sense coil 52 assigned the identifier s1, the position is represented by (X(s1), Y(s1), Z(s1)) and the orientation is represented by the vector NV(s1). Also, the position of the capsule endoscope 20 determined in advance is represented by (X(c), Y(c), Z(c)) and the orientation thereof is represented by NV(c).

First, when the distance between the sense coil assigned identifier s1 and the capsule endoscope 20, previously obtained by calculation as described above, is represented by r(s1), r(s1) can be represented by equation (1) shown below.

$$r_{(s1)} = \sqrt{\left(X_{(s1)} - X_{(c)}\right)^2 + \left(Y_{(s1)} - Y_{(c)}\right)^2 + \left(Z_{(s1)} - Z_{(c)}\right)^2} \qquad \ldots (1)$$

For sense coils 52 assigned other identifiers, the distance between each sense coil 52 and the capsule endoscope 20 can also be obtained by the position detection apparatus 50 performing computations based on equation (1) above.

Therefore, each time the position of the capsule endoscope 20 is detected, the position detection apparatus 50 sequentially selects a predetermined number of sense coils 52 close to the capsule endoscope 20 based on the distance obtained by the computation described above. Then, the position detection apparatus 50 outputs to the sense-coil selector 56 an instruction so that alternating current is output from the selected sense coils 52 to the sense-coil receiving circuit 57.

The method of selecting the sense coils 52 is not particularly limited. For example, as shown in Fig. 9, sense coils 52 opposing the drive coils 51 with the capsule endoscope 20 disposed therebetween may be selected, or as shown in Fig. 10, sense coils 52 that are disposed in mutually opposing planes adjacent to the plane in which the drive coils 51 are disposed may be selected.

Alternating currents that are induced in the sense coils 52 disposed on the three coil-supporting parts 58 may be selected by the sense-coil selector 56, as described above, or, without using the sense-coil selector 56, the number of coil-supporting parts 58 provided may be set beforehand to either one or two, as shown in Figs. 9 and 10.

Next, the operation of the magnetic induction apparatus 70 will be described.

As shown in Fig. 1, in the magnetic induction apparatus 70, first, the operator inputs a guidance direction for the capsule endoscope 20 to the rotation-magnetic-field control circuit 73 via the input device 74. In the rotation-magnetic-field control circuit 73, the orientation and rotation direction of a parallel magnetic field to be applied to the capsule endoscope 20 are determined based on the input guidance direction and the orientation (rotation axis direction) of the capsule endoscope 20 input from the position detection apparatus 50.

Then, to form the orientation of the parallel magnetic field, the required intensity of the magnetic fields produced by the Helmholtz coils 71X, 71Y, and 71Z are calculated, and the electrical currents required to produce these magnetic fields are calculated.

The electric current data supplied to the individual Helmholtz coils 71X, 71Y, and 71Z is output to the corresponding Helmholtz coil drivers 72X, 72Y, and 72Z, amplification control of the currents is carried out based on the input data, and the currents are supplied to the corresponding Helmholtz coils 71X, 71Y, and 71Z.

The Helmholtz coils 71X, 71Y, and 71Z to which the currents are supplied produce magnetic fields according to the respective current values, and by combining these magnetic fields, a parallel magnetic field having the magnetic field orientation determined by the rotation-magnetic-field control circuit 73 is produced.

The driving magnet 45 is provided in the capsule endoscope 20 and, as described later, the orientation (rotation axis direction) of the capsule endoscope 20 is controlled based on the force acting on the driving magnet 45 and the parallel magnetic field described above. Also, by controlling the rotation period of the parallel magnetic field to be about 0 Hz to a few Hz and controlling the rotation direction of the parallel magnetic field, the rotation direction about the rotation axis of the capsule endoscope 20 is controlled, and the direction of movement and the moving speed of the capsule endoscope 20 are controlled.

Next, the operation of the capsule endoscope 20 will be described.

As shown in Fig. 5, in the capsule endoscope 20, first infrared light is irradiated onto the infrared sensor 47 of the switch unit 46, and the switch unit 46 outputs a signal to the signal processing unit 34. When the signal processing unit 34 receives the signal from the switch unit 46, electrical current is supplied from the battery 39 to the image sensor 31, the LEDs 33, the radio device 35, and the image processing unit 34 itself, which are built into the capsule endoscope 20, and they are turned on.

The image sensor 31 images a wall surface inside the passage in the body cavity of the subject 1, which is illuminated by the LEDs 33, converts this image into an electrical signal, and outputs it to the signal processing unit 34. The signal processing unit 34 compresses the input image, temporarily stores it, and outputs it to the radio device 35. The compressed image signal input to the radio device 35 is transmitted to the image display device 80 as electromagnetic waves.

The capsule endoscope 20 can move towards the front end portion 23 or the rear end portion 24 by rotating the rotation axis R by means of the helical part 25 provided on the outer circumference of the outer casing 21. The direction of motion is determined by the rotation direction about the rotation axis R and the direction of rotation of the helical part 25.

Next, the operation of the image display device 80 will be described.

As shown in Fig. 1, in the image display device 80, first the image receiving circuit 81 receives the compressed image signal transmitted from the capsule endoscope 20, and the image signal is output to the display unit 82. The compressed image signal is reconstructed in the image receiving circuit 81 or the display unit 82, and is displayed by the display unit 82.

Also, the display unit 82 performs rotation processing on the image signal in the rotation direction of the reverse direction of the capsule endoscope 20 based on the rotation-phase data of the capsule endoscope 20, which is input from the rotation-magnetic-field control circuit 73, and displays it.

With the configuration described above, alternating magnetic fields are applied to the magnetic induction coil 42 of the capsule endoscope 20 from three or more different directions that are linearly independent. Therefore, it is possible to produce induced magnetism in the magnetic induction coil 42 due to alternating magnetic fields from at least one direction, irrespective of the orientation of the magnetic induction coil 42.

As a result, it is always possible to produce induced magnetism field in the magnetic induction coil 42, irrespective of the orientation (axial direction of the rotation axis R) of the capsule endoscope 20; therefore an advantage is afforded in that it is possible to always detect the induced magnetism by the sense coils 52, which allows the position thereof to always be detected with accuracy.

Also, since the sense coils 52 are disposed in three different directions with respect to the capsule endoscope 20, induced magnetism of detectable intensity acts on the sense coils 52 disposed in at least one direction of the sense coils 52 disposed in the three directions, which allows the sense coils 52 to always detect the induced magnetism, irrespective of the position at which the capsule endoscope 20 is disposed.

Furthermore, since the number of sense coils 52 disposed in one direction, as mentioned above, is nine, although a total of six pieces of information are determined by calculation, that is, the X, Y, and Z coordinates of the capsule endoscope 20, the rotation phases $\phi$ and $\theta$ about two axes orthogonal to each other and orthogonal to the rotation axis R of the capsule endoscope 20, and the intensity of the induced magnetism, a sufficient number of inputs can be obtained.

By making the frequency of the AC magnetic field the same as the frequency at which the resonance circuit 43 resonates (the resonance frequency), it is possible to produce induced magnetism with an amplitude that is large compared to the case where another frequency is used. Since the amplitude of the induced magnetism is large, the sense coils 52 can easily detect the induced magnetism, which makes it easy to detect the position of the capsule endoscope 20.

Also, since the frequency of the AC magnetic field sweeps over a frequency range in the vicinity of the resonance frequency, even if the resonance frequency of the resonance circuit 43 changes due to variations in the environmental conditions (for example, the temperature conditions) or even if there is a shift in the resonance frequency due to individual differences in the resonance circuit 43, it is possible to bring about resonance in the resonance circuit 43 so long as the changed resonance frequency or the shifted resonance frequency is included in the frequency range mentioned above.

Since the position detection apparatus 50 selects an output of the sense coil 52 that detects induced magnetism with a higher intensity by means of the sense-coil selector 56, it is possible to reduce the volume of information that the position detection apparatus 50 must calculate and process, which allows the computational load to be reduced. At the same time, since it is possible to simultaneously reduce the amount of computational processing, the time required for computation can be shortened.

By selecting the sense coil 52 based on the distance determined by computation, as described above, it is possible to perform position detection with superior accuracy. Furthermore, outputs from sense coils 52 having a poor S/N ratio can be excluded. Also, it is possible to reduce the amount of computation in the position detection apparatus 50, which allows cost-effective hardware to be constructed.

Since the drive coils 51 and the sense coils 52 are located at positions opposing each other on either side of the operating region of the capsule endoscope 20, the drive coils 51 and the sense coils 52 can be positioned so that they do not interfere with each other in terms of their construction.

By controlling the orientation of the parallel magnetic fields acting on the drive magnet 45 built into the capsule endoscope 20, it is possible to control the orientation of the force acting on the drive magnet 45, which allows the direction of motion of the capsule endoscope 20 to be controlled. Since it is possible to detect the position of the capsule endoscope 20 at the same time, the capsule endoscope can be guided to a predetermined position, and therefore, an advantage is afforded in that it is possible to accurately guide the capsule endoscope based on the detected position of the capsule endoscope 20.

By controlling the intensities of the magnetic fields produced by the three pairs of Helmholtz coils 71X, 71Y, and 71Z that are disposed to face each other in mutually orthogonal directions, the orientations of the parallel magnetic fields produced inside the Helmholtz coils 71X, 71Y, and 71Z can be controlled in a predetermined direction. Accordingly, a parallel magnetic field in a predetermined orientation can be applied to the capsule endoscope 20, and it is possible to move the capsule endoscope 20 in a predetermined direction.

Since the drive coils 51 and the sense coils 52 are disposed in the periphery of the space at the inner sides of the Helmholtz coils 71X, 71Y, and 71Z, which is the space in which the subject 1 can be positioned, the capsule endoscope 20 can be guided to a predetermined location in the body of the subject 1.

By rotating the capsule endoscope 20 about the rotation axis R, the helical part 25 produces a force that propels the capsule endoscope 20 in the axial direction of the rotation axis. Since the helical part 25 produces the propulsion force, it is possible to control the direction of the propulsion force acting on the capsule endoscope 20 by controlling the direction of rotation about the rotation axis R of the capsule endoscope 20.

Since the image display device 80 performs processing on image to be displayed to rotate it in the direction of rotation of the capsule endoscope 20 or the reverse direction, based on information on the rotation-phase about the rotational axis R of the capsule endoscope 20, it is possible to display on the display unit 82 an image that is always fixed at a predetermined rotational phase, in other words, an image in which the capsule endoscope 20 appears to travel along the rotation axis R without rotating about the rotation axis R, regardless of the rotational phase of the capsule endoscope 20.

Accordingly, when the capsule endoscope 20 is guided while the operator visually observes the image displayed on the image display unit 82, showing the image displayed in the manner described above as a predetermined rotational-phase image makes it easier for the operator to view and also makes it easier to guide the capsule endoscope 20 to a predetermined location, compared to the case where the displayed image is an image that rotates along with the rotation of the capsule endoscope 20.

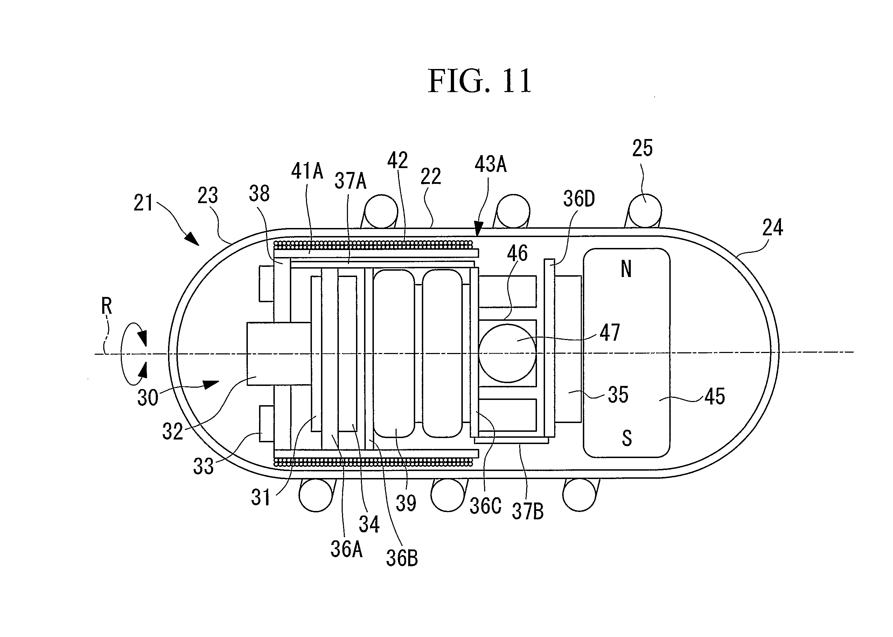

The construction of the capsule endoscope is not limited to the construction shown in Fig. 5; for example, it may have the construction shown in Fig. 11.

In a capsule endoscope 20A explained in Fig. 11, since the configuration of the induced-magnetism generator is different and the position of other devices is different, these are both described and the rest of the description is omitted.

Inside the outer casing 21 of the capsule endoscope 20A, the lens group 32, the LEDs 33, the image sensor 31, the signal processing unit 34, the battery 39, the switch unit 46, the radio device 35, and the driving magnet 45 are disposed in sequence from the front end portion 23.

The induced-magnetism generator 43A is disposed between the outer casing 21 and the battery 39 and so on, and is disposed so as to cover the components from the support member 38 of the LEDs 33 to the battery 39.

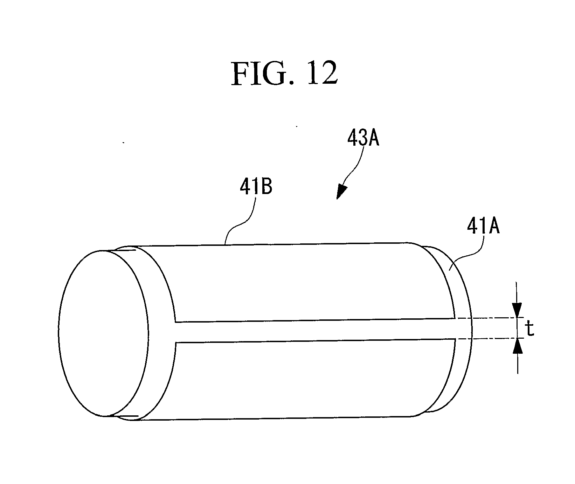

As shown in Figs. 11 and 12, the induced-magnetism generator 43A is formed of a core member 41A formed in the shape of a cylinder whose central axis is substantially coincident with the rotation axis R; an magnetic induction coil 42 disposed on the outer circumferential part of the core member 41A; a permalloy film 41B disposed between the core member 41A and the magnetic induction coil 42; and a capacitor (not shown in the figure) that is electrically connected to the magnetic induction coil 42 and that constitutes the resonance circuit 43.

As shown in Fig. 12, the permalloy film 41B is formed of a magnetic material in the form of a sheet. Also, when wrapping the permalloy film 41B around the core member 41A, a gap t is formed.

By disposing the permalloy film 41B between the core member 41A and the magnetic induction coil 42 in this way, it is possible to increase the intensity of the induced magnetism produced in the induced-magnetism generator 43A.

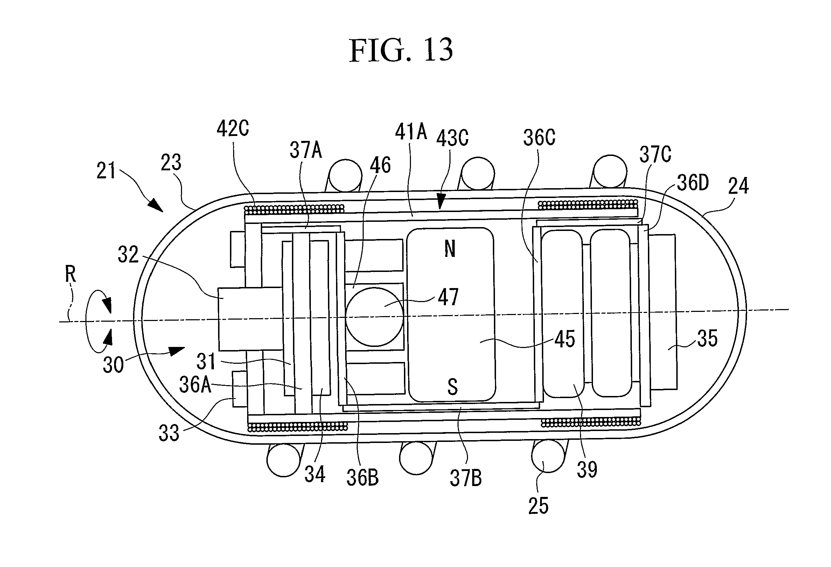

Also, in addition to the configurations shown in Figs. 5 and 11, the configuration of the capsule endoscope may be as shown in Fig. 13.

In a capsule endoscope 20B portrayed in Fig. 13, since the configuration of the induced-magnetism generator is different and the position of the other elements is different, these are both described, and the rest of the description shall be omitted.

Inside the outer casing 21 of the capsule endoscope 20B, the lens group 32, the LEDs 33, the image sensor 31, the signal processing unit 34, the switch unit 46, the driving magnet 45, the battery 39, and the radio device 35 are disposed in sequence from the front end portion 23.

An induced-magnetism generator 43C is disposed between the outer casing 21 and the battery 39 and the like, and is disposed so as to cover the elements from the support member 38 of the LEDs 33 to the battery 39.

As shown in Figs. 12 and 13, the induced-magnetism generator 43C is formed of a core member 41A formed in the shape of a cylinder whose central axis is substantially coincident with the rotation axis R; an magnetic induction coil 42C disposed on the outer circumferential part of the core member 41A; a permalloy film 41B disposed between the core member 41A and the magnetic induction coil 42C; and a capacitor (not shown in the figure) that is electrically connected to the magnetic induction coil 42C and that constitutes the resonance circuit 43.

The magnetic induction coil 42C is sparsely wound at the region where the driving magnet 45 is disposed and is densely wound at the front end portion 23 side and at the rear end portion 24 side.

As described above, when detecting the position and orientation of the capsule endoscope 20, an induced electromotive force is generated in the sense coils 52 due to both the alternating magnetic fields generated by the drive coils 51 and the induced magnetic field generated by the magnetic induction coil 42, and an amplitude value of the AC voltage may be extracted therefrom. In addition, it is also possible for an induced electromotive force to be initially generated in the sense coils 52 due to the AC magnetic field described above and the amplitude value of the AC voltage extracted therefrom to be stored in a storage unit of the position detection apparatus 50; thereafter, by producing an electromotive force due to both the AC magnetic field and the induced magnetic field, extracting an amplitude value of the AC voltage, and subtracting the amplitude value stored in the storage from this amplitude value, only an amplitude value produced by the induced magnetic field may be extracted.

With this method, by calculating the difference between the output of the sense coils 52 that receive the AC magnetic field and the induced magnetic field and the output of the sense coils 52 that receive only the AC magnetic field, which is stored in the storage unit described above, it is possible to easily determine only the output of the induced magnetic field from the output of the sense coils 52 receiving the AC magnetic field and the induced magnetic field.

As the predetermined number of sense coils 52 to be selected, six or more is preferable, as described above; by selecting more than six, it is possible to exclude the effects of noise included in the outputs of the sense coils 52. Also, by making the number of sense coils to be selected ten or less, the amount of calculation in the position detection apparatus 50 can be prevented from becoming excessively large.

Furthermore, a predetermined number of sense coils 52 to be selected may be set in advance, as described above; it is also possible to set a predetermined threshold value in advance and to use only sense coils 52 with a distance shorter than the predetermined threshold value.

By doing so, only the outputs of sense coils 52 having reliability can be extracted, which allows position calculation with higher accuracy to be realized.

First Modification of First Embodiment

Next, a first modification of the first embodiment of the present invention will be described.

The basic configuration of the capsule endoscope guidance system according to the modification of this embodiment is the same as that in the first embodiment; however, the method of detecting the position of the capsule endoscope is different. Thus, in this modification, only the position detection method of the capsule endoscope shall be described and the description of the magnetic induction apparatus and so forth shall be omitted.

In this modification, the selection method of the sense coils 52 to be used in estimation of the position and orientation of the capsule endoscope 20 by means of the position detection apparatus will be described.

In this modification, the intensity of the magnetic field created by the magnetic induction coil 42, which is installed in the capsule endoscope 20, is calculated for the respective sense coils 52 by the method shown below, and the sense coils 52 are selected based on the obtained magnetic field intensity.

Since the position detection of the magnetic induction coil 42 is the same as that in the first embodiment, a description thereof is omitted.

First, the position detection apparatus 50 calculates the magnitude and orientation of the magnetic field formed at the position where the magnetic induction coil 42 is disposed by the drive coils 51 based on the position of the capsule endoscope 20 (the magnetic induction coil 42), which is determined in advance.

More concretely, it is obtained by performing path integration of the current flowing in the drive coils 51 based on the Biot-Savart law. With this calculation, it is possible to obtain the magnetic field which the drive coils 51 produce at the position of the capsule endoscope 20 (the magnetic induction coil 42) determined in advance, and the obtained magnetic field is represented by a vector Bdv(c).

A current is induced in the magnetic induction coil 42 by the magnetic field Bdv(c) described above, and an induced magnetic field is produced by this induction. At this time, if the magnetic induction coil 42 is considered as a magnetic dipole having a dipole moment M(c), M(c) is expressed by equation (2) below, using Bdv(c) and Nv(c).

$$M_{(c)} = A1 \times \left( Bdv_{(c)} \cdot Nv_{(c)} \right) \quad \cdots (2)$$

Here, A1 is a constant determined by the properties of the magnetic induction coil, and "·" indicates the inner product of two vectors.

Then, the magnetic field Bv that the magnetic induction coil 42 produces at the position of each sensor coil 52, assuming it is at the position of the capsule endoscope 20 determined in advance, can be expressed by equation (3) below. Equation (3) shows an equation in which the magnetic field Bv(s1) produced at the sense coil 52 that is assigned the identifier s1 is obtained.

$$Bv_{(1)} = \frac{\mu_0}{4\pi} \times M_{(c)} \times \frac{3 rv_{(1)} \times \left( rv_{(1)} \cdot Nv_{(c)} \right) - r_{(1)}^{\,2} \times Nv_{(c)}}{r_{(1)}^{\,5}} \quad \cdots (3)$$

Here, $\mu 0$ is the magnetic permeability of a vacuum.

That is, by performing the calculations of Equations (2) and (3) shown above, the position detection apparatus 50 calculates the size of the magnetic field (magnetic field intensity) B that the magnetic induction coil 42 produces at the position of each sense coil 52.

Next, based on the magnetic field intensity produced at the sense coils 52, the position detection apparatus 50 selects a predetermined number of the sense coils 52 for which the magnetic field intensity B is large, and outputs to the sense-coil selector 56 an instruction for outputting an alternating current from the selected sense coils 52 to the sense-coil receiving circuit 57. Then, the position detection apparatus 50 calculates the position and orientation of the capsule endoscope 20 (magnetic induction coil 42) at the next calculation time, based on the outputs of the selected sense coils 52.

As the predetermined number of sense coils 52 to be selected, a number from six to ten, inclusive, is preferable, as described above.

With such a configuration, even if the outputs from all (27 in this embodiment) sense coils 52 are not measured, since suitable sense coils 52 are selected for use in calculating the position and orientation of the magnetic induction coil 42, the efficiency of computing the position and orientation can be improved.

As described above, the position detection apparatus 50 may select a predetermined number of the sense coils 52 in turn having a large magnetic field intensity B, or only the sense coils 52 in which a magnetic field B larger than a predetermined threshold value is formed may be selected.

By selecting the sense coils 52 in this way, even if the outputs from all sense coils 52 are not measured, it is possible to select suitable sense coils 52 for use in calculation of the position and orientation of the magnetic induction coil 42, and in addition, since only the outputs of the sense coils 52 having superior output characteristics can be used, it is possible to further improve the efficiency of calculating the position and orientation.

Second Modification of First Embodiment

Next, a second modification of the first embodiment of the present invention will be described.

The basic configuration of the capsule endoscope guidance system according to the modification of this embodiment is the same as in the first embodiment; however, the position detection method of the capsule endoscope is different from that in the first embodiment. Therefore, in this modification, only the position detection method of the capsule endoscope shall be described, and a description of the magnetic induction apparatus and the like shall be omitted.

The selection method of the sense coils 52 to be used in estimating the position and the orientation of the capsule endoscope 20 by means of the position detection apparatus in this modification will be described.

In this modification, the output of each sense coil is calculated based on the magnetic field produced at the position of each sense coil 52 by the magnetic induction coil 42, which is determined by the second modification described above, and the sense coils 52 are selected based on the output obtained.

First, based on the magnetic field produced at the position of each sense coil 52 by the magnetic induction coil 42, which is determined in advance, the position detection apparatus 50 performs a predetermined calculation to determine the output of the sense coils 52.

More concretely, for example, when the output from the sense coil assigned the identifier S1 is defined as V(s1), V(s1) is represented by equation (4) shown below.

$$V_{(s1)} = A2 \times \left( Bv_{(s1)} \cdot Nv_{(s1)} \right) \qquad \ldots (4)$$

Here, A2 is a proportionality constant determined by the properties of the sense coil.

The position detection apparatus 50 estimates the output of each sense coil 52 by calculation based on equation (4) shown above, and selects a predetermined number of the sense coils in turn for which the estimated output V is large. The position detection apparatus 50 outputs to the sense-coil selector 56 an instruction for outputting an alternating current from the selected sense coils 52 to the sense-coil receiving circuit 57.

Then, at the next calculation time, the position detection apparatus 50 calculates the position and the orientation of the capsule endoscope 20 (the magnetic induction coil 42) based on the outputs of the selected sense coils 52.

As the predetermined number of sense coils 52 to be selected, a number from six to ten, inclusive, is preferable, as described above.

With such a configuration, even if the outputs V from all sense coils 52 are not measured, since it is possible to select suitable sense coils 52 for use in calculation of the position and the orientation of the magnetic induction coil 42, the efficiency of calculation of the position and orientation can be improved.

As described above, the position detection apparatus 50 may select a predetermined number of the sense coils 52 in turn for which the outputs V of the sense coils 52 are large, or it may select only the sense coils 52 for which the outputs V are larger than a predetermined threshold value.

By selecting the sense coils 52 in this way, even if the outputs V of all sense coils 52 are not measured, it is possible to select suitable sense coils 52 for use in calculating the position and the orientation of the magnetic induction coil 42, and in addition, since it is possible to use only the outputs V of the sense coils 52 having superior output characteristics, the efficiency of calculation of the position and orientation can be further improved.

Second Embodiment

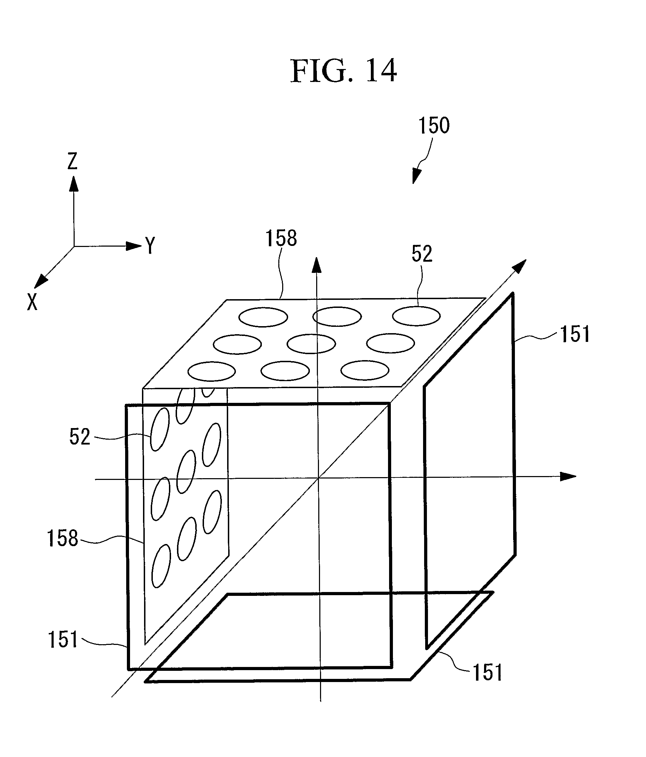

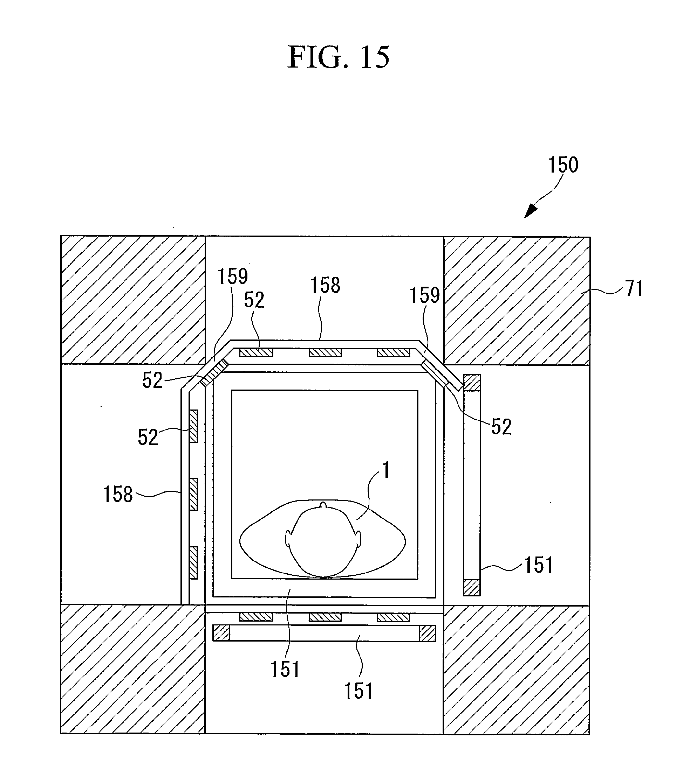

Next, a second embodiment of the present invention will be described with reference to Figs. 14 and 15.

The basic configuration of the capsule endoscope guidance system according to this embodiment is the same as that in the first embodiment; however, the configuration of the position detection apparatus is different from that in the first embodiment. Therefore, in this embodiment, only the vicinity of the position detection apparatus will be described using Figs. 14 and 15, and the description of the magnetic induction apparatus and the like will be omitted.

Fig. 14 is a schematic diagram showing the placement of drive coils and sense coils of the position detection apparatus.

Since the components other than the drive coils and the sense coils of the position detection apparatus are the same as in the first embodiment, a description thereof shall be omitted.

As shown in Fig. 14, regarding the drive coils (driving coils) 151 and sense coils 52 of a position detection apparatus 150, the drive coils 151 are disposed orthogonal to the X, Y, and Z axes, respectively, and the sense coils 52 are disposed on two planar coil-supporting members 158 that are orthogonal to the Y and Z axes, respectively.

As shown in the figure, rectangular coils may be used as the drive coils 151, or Helmholtz coils may be used.

As shown in Fig. 14, in the position detection apparatus 150 having the configuration described above, the orientations of the alternating magnetic fields that the drive coils 151 produce are parallel to the X, Y, and Z axial directions, and have a mutually orthogonal relationship, that is, they are linearly independent.

With this configuration, it is possible to cause the alternating magnetic fields from the mutually orthogonal directions, which are linearly independent, to be applied to the magnetic induction coil 42 in the capsule endoscope 20. Therefore, induced magnetism is easier to generate in the magnetic induction coil 42 compared to the first embodiment, regardless of the orientation of the magnetic induction coil 42.

Also, since the drive coils 151 are disposed so as to be substantially orthogonal to each other, selection of the drive coils by the drive-coil selector 55 is simplified.

The sense coils 52 may be disposed on the coil-support members 158, which are perpendicular to the Y and Z axis, as described above, or, as shown in Fig. 15, they may be provided on inclined coil-support members 159 that are disposed above the operating region of the capsule endoscope 20.

By positioning them in this manner, the sense coils 52 can be positioned without interfering with the subject 1.

Third Embodiment

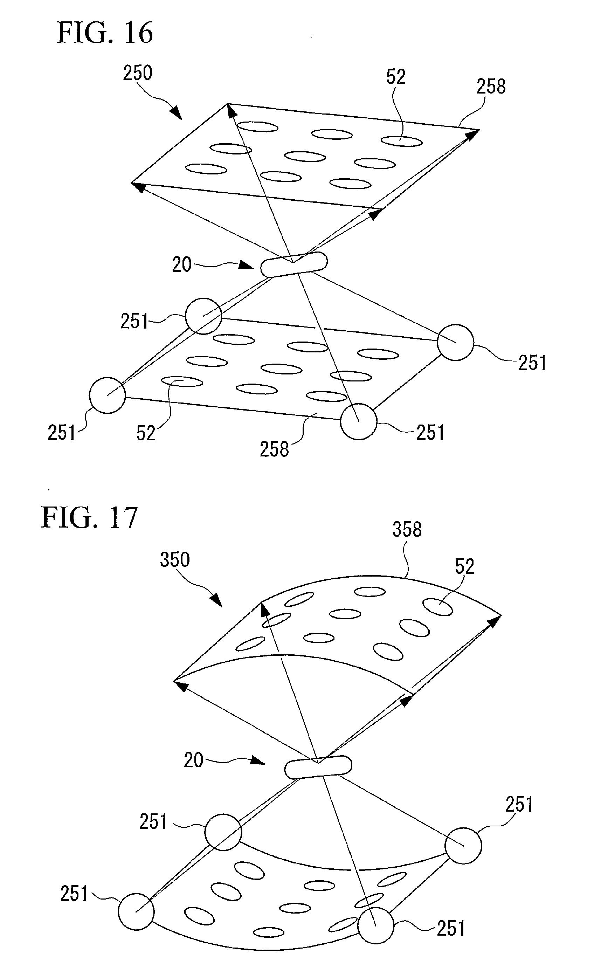

Next, a third embodiment of the present invention will be described with reference to Fig. 16.

The basic configuration of the capsule endoscope guidance system according to this embodiment is the same as that in the first embodiment; however, the configuration of the position detection apparatus is different from that in the first embodiment. Therefore, in this embodiment, only the vicinity of the position detection apparatus will be described using Fig. 16, and a description of the magnetic induction apparatus and the like will be omitted.

Fig. 16 is a schematic diagram showing the positioning of drive coils and sense coils in the position detection apparatus.

Since the components other than the drive coils and the sense coils of the position detection apparatus are the same as in the first embodiment, a description thereof shall be omitted.

Regarding drive coils (driving coils) 251 and sense coils 52 of a position detection apparatus 250, as shown in Fig. 16, four drive coils 251 are disposed in the same plane, and the sense coils 52 are disposed on a planar coil-supporting member 258, which is disposed at a position opposite the position where the drive coils 251 are located, and on a planar coil-supporting member 258, which is disposed at the same side where the drive coils 251 are located, the operating region of the capsule endoscope 20 being disposed therebetween.

The orientations of the alternating magnetic fields that the drive coils 251 produce are disposed so as to be linearly independent of each other, as indicated by the arrows in the figure.

According to this configuration, one of the two coil-supporting members 258 is located close with respect to the capsule endoscope 20, even when the capsule endoscope 20 is located in a nearby region or a distant region with respect to the drive coils 251. Accordingly, a signal of sufficient intensity can be obtained from the sense coils 52 when determining the position of the capsule endoscope 20.

Modification of Third Embodiment

Next, a modification of the third embodiment of the present invention will be described with reference to Fig. 17.

The basic configuration of the capsule endoscope guidance system of this modification is the same as that in the third embodiment; however, the configuration of the position detection apparatus is different from that in the third embodiment. Therefore, in this embodiment, only the vicinity of the position detection apparatus will be described using Fig. 17, and a description of the magnetic induction apparatus and the like will be omitted.

Fig. 17 is a schematic diagram showing the positioning of drive coils and sense coils of the position detection apparatus.

Since the components other than the drive coils and the sense coils of the position detection apparatus are the same as in the third embodiment, a description thereof is omitted here.

Regarding drive coils 251 and sense coils 52 of the position detection apparatus 350, as shown in Fig. 17, four drive coils 251 are disposed in the same plane, and the sense coils 52 are disposed on a curved coil-supporting member 358, which is disposed at a position opposite the position where the drive coils 251 are located, and on a curved coil-supporting member 358, which is disposed at the same side where the drive coils 251 are located, the operating region of the capsule endoscope 20 being disposed therebetween.

The coil-supporting members 358 are formed in a curved shape that is convex towards the outer side relative to the operating region of the capsule endoscope 20, and the sense coils 52 are disposed over the curved surfaces.

The shape of the coil-supporting members 358 may be curved surfaces that are convex towards the outer side with respect to the operating region, as described above, or they may be any other shape of curved surface and are not particularly limited.

With the configuration described above, since degree of freedom of positioning the sense coils 52 is improved, it is possible to prevent the sense coils 52 from interfering with the subject 1.

Fourth Embodiment

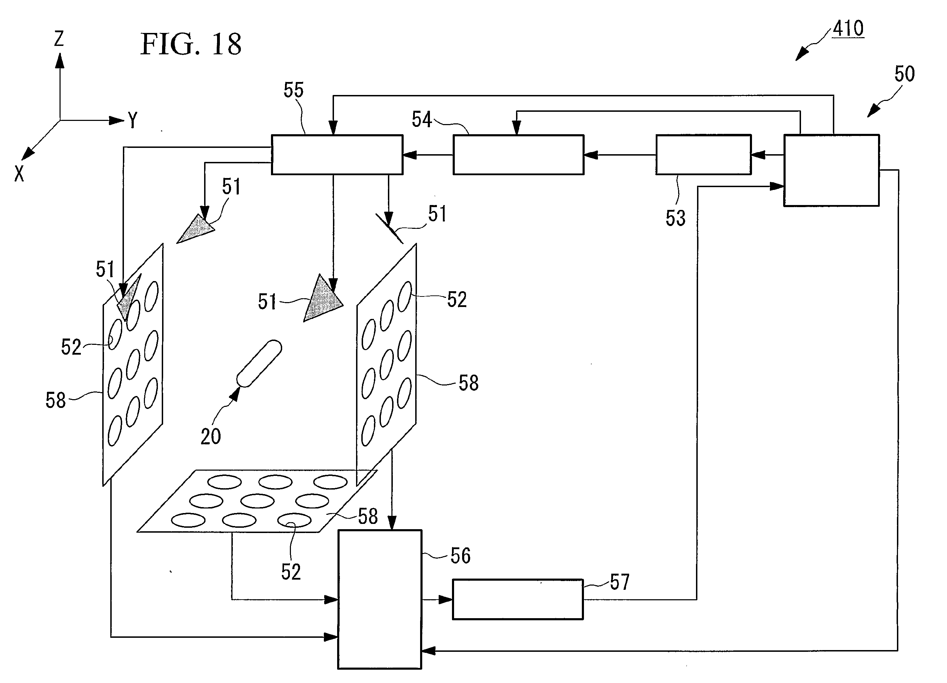

A position detection system for a capsule endoscope according to the present invention will be described below with reference to Fig. 18.

Fig. 18 shows an outline of the position detection system for a capsule endoscope according to the present invention.

A position detection system 410 for a capsule endoscope according to the present invention is formed of only the position detection apparatus 50 of the capsule endoscope guidance system 10 described above. Therefore, since the components, operation, and advantages of the position detection system 410 for a capsule endoscope are the same as those in the capsule endoscope guidance system 10, Fig. 18 is shown and a description thereof is omitted.

The technical field of the present invention is not limited to the embodiments described above, various modifications may be applied within the scope thereof without departing from the gist of the invention.

For example, in the embodiments described above, as the medical device, a description has been given of a device using a capsule endoscope that captures images of the interior of a body cavity of a subject; however, the invention is not limited to such a device using a capsule endoscope. It can be applied to various other types of medical device, such as a medical device that discharges a drug inside the body cavity of the subject, a medical device provided with a sensor for acquiring data on the interior of the body cavity; a medical device that can be left inside the body cavity for a long period of time, a medical device in which wiring lines for exchanging information and the like are connected to the exterior, and so forth.

According to the medical-device position detection system according to the present invention, by making magnetism act on a magnetic induction coil from at least three different directions, it is possible to always generate induced magnetism in the magnetic induction coil, regardless of the orientation of the medical device, and therefore, an advantage is afforded in that it is possible to always accurately detect the position thereof.

Furthermore, according to the medical-device guidance system of the present invention, based on the position of the medical device detected by the medical-device position detection system according to the present invention described above, an advantage is afforded in that it is possible to accurately guide the medical device.

**Claims**

1. A position detection system (50) for a medical device (20) that is inserted into a body of a subject, comprising:

   a magnetic induction coil (42) installed in the medical device (20); and
   a magnetic sensor (52), disposed outside an operating region of the medical device (20), that is configured to detect induced magnetism generated by the magnetic induction coil (42),
**characterized by** further comprising at least one driving coil (151), disposed outside the operating region of the medical device (20), that is configured to generate induced magnetism in the magnetic induction coil (42), wherein, when the medical device (20) is disposed at individual positions in the operating region of the medical device (20), the at least one driving coil (151) is configured to cause magnetism to act on the magnetic induction coil (42) from three or more different directions, and
among the three or more directions in which the magnetism acts, at least one of the directions is arranged to be a direction intersecting a plane formed by the other two directions.

2. The position detection system (50) for a medical device (20) according to Claim 1, wherein
a plurality of magnetic sensors (52) are provided, and
the plurality of magnetic sensors (52) are disposed facing the operating region of the medical device (20) in a plurality of directions.

3. The position detection system (50) for a medical device (20) according to Claim 1, wherein
the driving coil (151) is configured to generate induced magnetism in the magnetic induction coil (42) over a frequency range near a resonance frequency of a resonance circuit (43) that includes the magnetic induction coil (42).

4. The position detection system (50) for a medical device (20) according to Claim 2, further comprising:

a magnetic-sensor selection unit (56) that is configured to selectively use the output of a sensor detected to have a high intensity of the induced magnetism among the induced magnetisms detected by the plurality of magnetic sensors (52).

5. The position detection system (50) for a medical device (20) according to Claim 1, wherein
the driving coil (151) and the magnetic sensor (52) are disposed at opposing positions on either side of the operating region of the medical device (20).

6. The position detection system (50) for a medical device (20) according to claim 1, said system being adapted to repeadtedly detect a position and orientation of the medical device, said system
further comprising:

a plurality of magnetic sensors (52);
a position calculating device (50) configured to determine, by repeated calculation, the position and orientation of the medical device based on outputs of the magnetic sensors (52); and
a magnetic-sensor selection unit (56) configured to select a magnetic sensor to use from the plurality of magnetic sensors (52) to determine the position and orientation of the medical device (20) in the calculation in the position calculating device, wherein
the plurality of magnetic sensors (52) is configured to repeatedly detect the magnetic field generated by the coil (42).

7. The position detection system (50) for a medical device (20) according to Claim 6, further comprising:

an alternating magnetic field generating device, provided outside the body of the subject, configured to generate an alternating magnetic field;
a storage unit configured to store an output indicating a magnetic field intensity that the magnetic sensor receives; and
a change detection unit configured to determine the amount of change in the output of the magnetic sensor by subtracting the output stored in the storage unit from the output of the magnetic sensor;
wherein the coil (42) is a magnetic induction coil configured to generate an induced magnetic field by receiving the magnetic field generated by the alternating magnetic field generating device;
the output of a magnetic sensor, when the magnetic sensor received only the alternating magnetic field, is stored in the storage unit; and
the change detection unit is configured to determine the induced magnetic field generated by the coil by subtracting the output stored in the storage unit from the output of a magnetic sensor, when the magnetic sensor received the alternating magnetic field, and the induced magnetic field.

8. The position detection system (50) for a medical device (20) according to Claim 6, wherein

the position calculating device is configured to estimate the output of the plurality of magnetic sensors at the next calculation time based on the position and the orientation of the medical device determined by the calculation; and the magnetic-sensor selection unit (56) is configured to select the magnetic sensor to be used when determining the position and the orientation of the medical device at the next calculation time based on the estimated outputs of the plurality of magnetic sensors.

9. The position detection system (50) for a medical device (20) according to Claim 8, wherein the magnetic-sensor selection unit (56) is configured to sequentially select a predetermined number of the magnetic sensors for which the estimated outputs of the plurality of magnetic sensors are large.

10. The position detection system (50) for a medical device (20) according to Claim 8, wherein the magnetic-sensor selection unit (56) is configured to select the magnetic sensors for which the estimated outputs of the plurality of magnetic sensors are larger than a predetermined value.

11. The position detection system (50) for a medical device (20) according to Claim 6, wherein the position calculating device is configured to estimate the size and the orientation of the magnetic field that the coil forms at the positions of individual magnetic sensors based on the position and the orientation determined by the calculation; and the magnetic-sensor selection unit (56) is configured to select the magnetic sensor to be used in determining the position and the orientation of the medical device at the next calculation time based on the estimated outputs of the individual magnetic sensors.

12. The position detection system (50) for a medical device (20) according to Claim 11, wherein the magnetic-sensor selection unit (56) is configured to sequentially select a predetermined number of the magnetic sensors for which the intensities of the magnetic fields that the coil forms at the positions of the plurality of magnetic sensors are large.

13. The position detection system (50) according to Claim 6, wherein the position calculating device is configured to estimate the distance between each magnetic sensor and the coil based on the position and the orientation of the medical device determined by the calculation; and the magnetic-sensor selection unit (56) is configured to select the magnetic sensor to be used in determining the position and the orientation of the medical device at the next calculation time based on the estimated distance between each magnetic sensor and the coil.

14. The position detection system (50) for a medical device (20) according to Claim 13, wherein the magnetic-sensor selection unit (56) is configured to select the magnetic sensor for which the estimated distance between the magnetic sensor and the coil is smaller than a predetermined value.

15. The position detection system (50) according to Claim 1, wherein the medical device (20) is a capsule endoscope, and the longitudinal axis of the capsule endoscope and the direction of a central axis of the coil are made substantially the same.

16. A medical-device guidance system (10) comprising:

   the position detection system (50) according to Claim 1;
   a magnet (45) installed in the medical device (20);
   a magnetic-field generating unit (71), disposed outside the operating region of the medical device (20), that is configured to generate a magnetic field that is made to act on the magnet (45); and
   a magnetic-field-orientation control (73) unit configured to control the orientation of the magnetic field applied to the magnet (45) by the magnetic-field generating unit (71).

17. The medical-device guidance system (10) according to Claim 16, wherein the magnetic-field generating unit (71) includes three pairs of opposed frame-shaped electromagnets that are disposed in mutually orthogonal directions; a space in which the body of the subject can be positioned is provided inside the electromagnets; and the driving coil (151) and the magnetic sensors (52) are disposed in the periphery of the space in which the body of the subject can be positioned.

**18.** The medical-device guidance system (10) according to Claim 16, wherein
the magnetic-field generating unit is configured to generate a rotating magnetic field around the medical device (20); and
a spiral that converts rotary force about a longitudinal axis of the medical device (20) into a propulsion force in the direction of the longitudinal axis is provided on the outer surface of the medical device (20).

**19.** The medical-device guidance system (10) according to Claim 18, wherein
the medical device (20) includes an imaging unit (30) having an optical axis parallel to the longitudinal axis of the medical device (20); and
further comprising an image control unit that is configured to rotate the image captured by the imaging unit (30) in an opposite direction based on rotation information about the longitudinal axis of the medical device (20) by the magnetic-field-orientation control unit (73); and
a display unit (82) that is configured to display the image captured by the imaging unit (30) and/or the image rotated by the image control unit.

**20.** The medical-device guidance system (10) according to Claim 16, wherein
the medical device (20) is a capsule endoscope, and the longitudinal axis of the capsule endoscope and the direction of a central axis of the coil are made substantially the same.

**21.** The position detection system (50) for a medical device (20) according to Claim 1, further comprising:

a driving-coil selecting unit configured to selectively use a plurality of driving coils.

**22.** The position detection system (50) for a medical device (20) according to Claim 1, wherein
a plurality of driving coils (151) are disposed so as to be mutually orthogonal.

**23.** The medical-device guidance system (10) according to Claim 16, wherein
one of the driving coil (151) and the magnetic sensor (52) is disposed at the upper part of a space in which the subject can be positioned.


**Patentansprüche**

**1.** Positionsnachweissystem (50) für ein Medizinprodukt (20), das in einen Körper eines Patienten eingeführt wird, umfassend:

eine magnetische Induktionsspule (42), die in das Medizinprodukt (20) eingesetzt ist; und
einen Magnetsensor (52), der außerhalb eines Betriebsbereichs des Medizinprodukts (20) angeordnet ist und der dazu ausgebildet ist, einen von der magnetischen Induktionsspule (42) erzeugten induzierten Magnetismus zu erfassen,
**dadurch gekennzeichnet, dass** es ferner wenigstens eine Anregungsspule (151) umfasst, die außerhalb des Betriebsbereichs des Medizinprodukts (20) angeordnet und dazu ausgebildet ist, in der magnetischen Induktionsspule (42) einen induzierten Magnetismus zu erzeugen,
wobei, wenn sich das Medizinprodukt (20) an einzelnen Positionen im Betriebsbereich des Medizinprodukts (20) befindet, die wenigstens eine Anregungsspule (151) dazu ausgebildet ist, zu bewirken, dass Magnetismus aus drei oder mehr verschiedenen Richtungen auf die magnetische Induktionsspule (42) einwirkt, und
wobei unter den drei oder mehr Richtungen, in denen der Magnetismus wirkt, wenigstens eine der Richtungen so angeordnet ist, dass sie eine Richtung darstellt, die eine Ebene schneidet, die von den anderen beiden Richtungen gebildet wird.

**2.** Positionsnachweissystem (50) für ein Medizinprodukt (20) nach Anspruch 1, bei dem
eine Mehrzahl von Magnetsensoren (52) vorgesehen sind, und
die Mehrzahl von Magnetsensoren (52) so angeordnet sind, dass sie dem Betriebsbereich des Medizinprodukts (20) in einer Mehrzahl von Richtungen zugewandt sind.

**3.** Positionsnachweissystem (50) für ein Medizinprodukt (20) nach Anspruch 1, bei dem
die Anregungsspule (151) dazu ausgebildet ist, über einen Frequenzbereich, der nahe der Resonanzfrequenz eines die magnetische Induktionsspule (42) beinhaltenden Resonanzkreises (43) liegt, in der magnetischen Induktionsspule (42) einen induzierten Magnetismus zu erzeugen.

4.  Positionsnachweissystem (50) für ein Medizinprodukt (20) nach Anspruch 2, das ferner umfasst:

    eine Magnetsensor-Auswahleinheit (56), die dazu ausgebildet ist, wahlweise das Ausgangssignal eines Sensors zu verwenden, bei dem erfasst wurde, dass er unter den von der Mehrzahl von Magnetsensoren (52) erfassten induzierten Magnetismen eine hohe Intensität des induzierten Magnetismus aufweist.

5.  Positionsnachweissystem (50) für ein Medizinprodukt (20) nach Anspruch 1, bei dem
    die Anregungsspule (151) und der Magnetsensor (52) an gegenüberliegenden Positionen auf beiden Seiten des Betriebsbereichs des Medizinprodukts (20) angeordnet sind.

6.  Positionsnachweissystem (50) für ein Medizinprodukt (20) nach Anspruch 1, wobei das System wiederholt eine Position und eine Ausrichtung des Medizinprodukts zu erfassen vermag, wobei das System ferner umfasst:

    eine Mehrzahl von Magnetsensoren (52);
    eine Positionsberechnungsvorrichtung (50), die dazu ausgebildet ist, auf der Grundlage von Ausgangssignalen des Magnetsensors (52) durch wiederholte Berechnung die Position und die Ausrichtung des Medizinprodukts zu bestimmen; und
    eine Magnetsensor-Auswahleinheit (56), die dazu ausgebildet ist, aus der Mehrzahl von Magnetsensoren (52) einen zu Magnetsensor auszuwählen, der zu verwenden ist, um die Position und die Ausrichtung des Medizinprodukts (20) bei der Berechnung in der Positionsberechnungsvorrichtung zu bestimmen, wobei
    die Mehrzahl von Magnetsensoren (52) dazu ausgebildet ist, das von der Spule (42) erzeugte Magnetfeld wiederholt zu erfassen.

7.  Positionsnachweissystem (50) für ein Medizinprodukt (20) nach Anspruch 6, das ferner umfasst:

    eine Vorrichtung zum Erzeugen eines magnetischen Wechselfelds, die außerhalb des Körpers des Patienten vorgesehen und dazu ausgebildet ist, ein magnetisches Wechselfeld zu erzeugen;
    eine Speichereinheit, die dazu ausgebildet ist, ein Ausgangssignal zu speichern, das die Intensität eines vom Magnetsensor empfangenen Magnetfelds angibt; und
    eine Änderungserfassungseinheit, die dazu ausgebildet ist, den Betrag der Änderung im Ausgangssignal des Magnetsensors zu bestimmen, indem sie das in der Speichereinheit gespeicherte Ausgangssignal von dem Ausgangssignal des Magnetsensors subtrahiert;
    wobei die Spule (42) eine magnetische Induktionsspule ist, die dazu ausgebildet ist, ein induziertes Magnetfeld zu erzeugen, indem sie das durch die Vorrichtung zum Erzeugen eines magnetischen Wechselfelds erzeugte Magnetfeld empfängt,
    wenn der Magnetsensor nur das magnetische Wechselfeld empfangen hat, das Ausgangssignal des Magnetsensors in der Speichereinheit gespeichert wird; und
    die Änderungserfassungseinheit dazu ausgebildet ist, das von der Spule erzeugte induzierte Magnetfeld zu bestimmen, durch Subtraktion des in der Speichereinheit abgespeicherten Ausgangssignals vom Ausgangssignal eines Magnetsensors, wenn der Magnetsensor das magnetische Wechselfeld und das induzierte Magnetfeld empfangen hat.

8.  Positionsnachweissystem (50) für ein Medizinprodukt (20) nach Anspruch 6, bei dem
    die Positionsberechnungsvorrichtung dazu ausgebildet ist, auf der Grundlage der durch die Berechnung bestimmten Position und Ausrichtung des Medizinprodukts das Ausgangssignal der Mehrzahl von Magnetsensoren zum nächsten Berechnungszeitpunkt abzuschätzen; und
    die Magnetsensor-Auswahleinheit (56) dazu ausgebildet ist, auf der Grundlage der geschätzten Ausgangssignale der Mehrzahl von Magnetsensoren, den Magnetsensor auszuwählen, der zu verwenden ist, wenn die Position und die Ausrichtung des Medizinprodukts zum nächsten Berechnungszeitpunkt bestimmt wird.

9.  Positionsnachweissystem (50) für ein Medizinprodukt (20) nach Anspruch 8, bei dem
    die Magnetsensor-Auswahleinheit (56) dazu ausgebildet ist, der Reihe nach eine vorgegebene Anzahl von Magnetsensoren auszuwählen, für die die geschätzten Ausgangssignale der Mehrzahl von Magnetsensoren hoch sind.

10. Positionsnachweissystem (50) für ein Medizinprodukt (20) nach Anspruch 6, bei dem
    die Magnetsensor-Auswahleinheit (56) dazu ausgebildet ist, die Magnetsensoren auszuwählen, für die die geschätzten Ausgangssignale der Mehrzahl von Magnetsensoren höher sind als ein vorgegebener Wert.

11. Positionsnachweissystem (50) für ein Medizinprodukt (20) nach Anspruch 6, bei dem
die Positionsberechnungsvorrichtung dazu ausgebildet ist, auf der Grundlage der durch die Berechnung bestimmten Position und Ausrichtung die Größe und die Orientierung des von der Spule an den Positionen einzelner Magnetsensoren gebildeten Magnetfelds abzuschätzen; und
die Magnetsensor-Auswahleinheit (56) dazu ausgebildet ist, auf der Grundlage der geschätzten Ausgangssignale der einzelnen Magnetsensoren den Magnetsensor auszuwählen, der bei der Bestimmung der Position und der Ausrichtung des Medizinprodukts zum nächsten Berechnungszeitpunkt zu verwenden ist.

12. Positionsnachweissystem (50) für ein Medizinprodukt (20) nach Anspruch 11, bei dem
die Magnetsensor-Auswahleinheit (56) dazu ausgebildet ist, der Reihe nach eine vorgegebene Anzahl von Magnetsensoren auszuwählen, für die die Intensität der von der Spule an den Positionen der Mehrzahl von Magnetsensoren gebildeten Magnetfelder hoch ist.

13. Positionsnachweissystem (50) für ein Medizinprodukt (20) nach Anspruch 6, bei dem
die Positionsberechnungsvorrichtung dazu ausgebildet ist, auf der Grundlage der durch die Berechnung bestimmten Position und Ausrichtung des Medizinprodukts den Abstand zwischen jedem Magnetsensor und der Spule abzuschätzen; und
die Magnetsensor-Auswahleinheit (56) ausgebildet ist, auf der Grundlage des geschätzten Abstands zwischen jedem Magnetsensor und der Spule den Magnetsensor auszuwählen, der bei der Bestimmung der Position und der Ausrichtung des Medizinprodukts zum nächsten Berechnungszeitpunkt zu verwenden ist.

14. Positionsnachweissystem (50) für ein Medizinprodukt (20) nach Anspruch 13, bei dem die Magnetsensor-Auswahleinheit (56) dazu ausgebildet ist, den Magnetsensor auszuwählen, bei dem der geschätzte Abstand zwischen dem Magnetsensor und der Spule geringer ist als ein vorgegebener Wert.

15. Positionsnachweissystem (50) nach Anspruch 1, bei dem das Medizinprodukt (20) ein Kapselendoskop ist und die Längsachse des Kapselendoskops und die Richtung einer Mittelachse der Spule im Wesentlichen in Übereinstimmung gebracht werden.

16. Medizinprodukt-Führungssystem (10), umfassend:

   das Positionsnachweissystem (50) nach Anspruch 1;
   einen in das Medizinprodukt (20) eingebauten Magneten (45);
   eine Magnetfelderzeugungseinheit (71), die außerhalb des Betriebsbereichs des Medizinprodukts (20) angeordnet und dazu ausgebildet ist, ein Magnetfeld zu erzeugen, das erstellt wird, um auf den Magneten (45) einzuwirken; und
   eine Magnetfeldausrichtungssteuereinheit (73), die dazu ausgebildet ist, die Ausrichtung des durch die Magnetfelderzeugungseinheit (71) an den Magneten (45) angelegten Magnetfelds zu steuern.

17. Medizinprodukt-Führungssystem (10) nach Anspruch 16, bei dem
die Magnetfelderzeugungseinheit (71) drei Paar von einander gegenüberliegend angeordneten, rahmenförmigen Elektromagneten aufweist, die in orthogonaler Richtung zueinander angeordnet sind;
innerhalb der Elektromagnete ein Raum vorgesehen ist, in dem der Körper des Patienten positioniert werden kann; und
die Anregungsspule (151) und der Magnetsensor (52) am äußeren Rand des Raums angeordnet sind, in dem der Körper des Patienten positioniert werden kann.

18. Medizinprodukt-Führungssystem (10) nach Anspruch 16, bei dem
die Magnetfelderzeugungseinheit dazu ausgebildet ist, um das Medizinprodukt (20) ein rotierendes Magnetfeld zu erzeugen; und
eine Spirale auf der Außenfläche des Medizinprodukts (20) vorgesehen ist, die eine um die Längsachse des Medizinprodukts (20) wirkende Drehkraft in eine Antriebskraft in der Richtung der Längsachse umwandelt.

19. Medizinprodukt-Führungssystem (10) nach Anspruch 18, bei dem
das Medizinprodukt (20) eine Abbildungseinheit (30) umfasst, die eine optische Achse parallel zur Längsachse des Medizinprodukts (20) aufweist; und

ferner eine Bildsteuereinrichtung vorgesehen ist, die dazu ausgebildet ist, das von der Abbildungseinheit (30) aufgenommene Bild in eine entgegengesetzte Richtung zu drehen, auf der Grundlage von Informationen über die Längsachse des Medizinprodukts (20) von der Magnetfeldausrichtungssteuereinheit (73); und

eine Anzeigeeinheit (82), die dazu ausgebildet ist, das von der Abbildungseinheit (30) aufgenommene Bild und/oder das von der Bildsteuereinrichtung gedrehte Bild anzuzeigen.

20. Medizinprodukt-Führungssystem (10) nach Anspruch 16, bei dem
das Medizinprodukt (20) ein Kapselendoskop ist und die Längsachse des Kapselendoskops und die Richtung einer Mittelachse der Spule im Wesentlichen in Übereinstimmung gebracht werden.

21. Positionsnachweissystem (50) für ein Medizinprodukt (20) nach Anspruch 1, das ferner umfasst:

eine Anregungsspulen-Auswahleinheit, die dazu ausgebildet ist, eine Mehrzahl von Anregungsspulen wahlweise zu verwenden.

22. Positionsnachweissystem (50) für ein Medizinprodukt (20) nach Anspruch 1, bei dem
eine Mehrzahl von Anregungsspulen (151) so angeordnet werden, dass sie gegenseitig orthogonal sind.

23. Medizinprodukt-Führungssystem (10) nach Anspruch 16, bei dem
eine der Anregungsspulen (151) und der Magnetsensor (52) im oberen Teil eines Raumes angeordnet sind, in dem ein Patient positioniert werden kann.

**Revendications**

1.  Système (50) de détection de position pour un dispositif médical (20) qui est insérée dans un corps d'un sujet, comprenant :

une bobine d'induction magnétique (42) installée dans le dispositif médical (20) ; et
un capteur magnétique (52), disposé à l'extérieur d'une région de fonctionnement du dispositif médical (20), qui est configuré pour détecter le magnétisme induit généré par la bobine d'induction magnétique (42), **caractérisé en ce qu'**il comprend en outre au moins une bobine d'entraînement (151), disposée à l'extérieur de la région de fonctionnement du dispositif médical (20), qui est configurée pour générer un magnétisme induit dans la bobine d'induction magnétique (42),
dans lequel, lorsque le dispositif médical (20) est disposé à des positions individuelles dans la région de fonctionnement du dispositif médical (20), la au moins une bobine d'entraînement (151) est configurée pour amener le magnétisme à agir sur la bobine d'induction magnétique (42) à partir de trois directions différentes ou plus, et parmi les trois directions ou plus dans lesquelles le magnétisme agit, au moins l'une des directions est agencée pour être une direction croisant un plan formé par les deux autres directions.

2.  Système (50) de détection de position pour un dispositif médical (20) selon la revendication 1, dans lequel
une pluralité de capteurs magnétiques (52) sont prévus, et
la pluralité de capteurs magnétiques (52) sont disposés en regard de la région de fonctionnement du dispositif médical (20) dans une pluralité de directions.

3.  Système (50) de détection de position pour un dispositif médical (20) selon la revendication 1, dans lequel
la bobine d'entraînement (151) est configurée pour générer un magnétisme induit dans la bobine d'induction magnétique (42) sur une plage de fréquence proche d'une fréquence de résonance d'un circuit de résonance (43) qui inclut la bobine d'induction magnétique (42).

4.  Système (50) de détection de position pour un dispositif médical (20) selon la revendication 2, comprenant en outre :

une unité (56) de sélection de capteurs magnétiques qui est configurée pour utiliser sélectivement la sortie d'un capteur détectée comme présentant une intensité élevée de magnétisme induit parmi les magnétismes induits détectés par la pluralité de capteurs magnétiques (52).

5.  Système (50) de détection de position pour un dispositif médical (20) selon la revendication 1, dans lequel
la bobine d'entraînement (151) et le capteur magnétique (52) sont disposés à des positions opposées de chaque côté de la région de fonctionnement du dispositif médical (20).

**6.** Système (50) de détection de position pour un dispositif médical (20) selon la revendication 1, ledit système étant adapté pour détecter de manière répétée une position et une orientation du dispositif médical, ledit système comprenant en outre :

une pluralité de capteurs magnétiques (52) ;
un dispositif (50) de calcul de position configuré pour déterminer, par un calcul répété, la position et l'orientation du dispositif médical sur la base des sorties des capteurs magnétiques (52) ; et
une unité (56) de sélection de capteurs magnétiques configurée pour sélectionner un capteur magnétique à utiliser parmi la pluralité de capteurs magnétiques (52) pour déterminer la position et l'orientation du dispositif médical (20) lors du calcul dans le dispositif de calcul de position, dans lequel
la pluralité de capteurs magnétiques (52) sont configurés pour détecter de manière répétée le champ magnétique généré par la bobine (42).

**7.** Système (50) de détection de position pour un dispositif médical (20) selon la revendication 6, comprenant en outre :

un dispositif de génération de champ magnétique alternatif, prévu à l'extérieur du corps du sujet, configuré pour générer un champ magnétique alternatif ;
une unité de mémoire configurée pour mémoriser une sortie indiquant une intensité de champ magnétique que le capteur magnétique reçoit ; et
une unité de détection de changement configurée pour déterminer la quantité de changement dans la sortie du capteur magnétique en soustrayant la sortie mémorisée dans l'unité de mémoire de la sortie du capteur magnétique ;
dans lequel la bobine (42) est une bobine d'induction magnétique configurée pour générer un champ magnétique induit en recevant le champ magnétique généré par le dispositif de génération de champ magnétique alternatif ;
la sortie d'un capteur magnétique, lorsque le capteur magnétique a reçu uniquement le champ magnétique alternatif, est mémorisée dans l'unité de mémoire ; et
l'unité de détection de changement est configurée pour déterminer le champ magnétique induit généré par la bobine en soustrayant la sortie mémorisée dans l'unité de mémoire de la sortie d'un capteur magnétique, lorsque le capteur magnétique a reçu le champ magnétique alternatif, et le champ magnétique induit.

**8.** Système (50) de détection de position pour un dispositif médical (20) selon la revendication 6, dans lequel le dispositif de calcul de position est configuré pour estimer la sortie de la pluralité de capteurs magnétiques lors du calcul suivant sur la base de la position et de l'orientation du dispositif médical déterminées par le calcul ; et l'unité (56) de sélection de capteurs magnétiques est configurée pour sélectionner le capteur magnétique devant être utilisé lors de la détermination de la position et de l'orientation du dispositif médical lors du calcul suivant sur la base des sorties estimées de la pluralité de capteurs magnétiques.

**9.** Système (50) de détection de position pour un dispositif médical (20) selon la revendication 8, dans lequel l'unité (56) de sélection de capteurs magnétiques est configurée pour sélectionner séquentiellement un nombre prédéterminé de capteurs magnétiques pour lesquels les sorties estimées de la pluralité de capteurs magnétiques sont importantes.

**10.** Système (50) de détection de position pour un dispositif médical (20) selon la revendication 8, dans lequel l'unité (56) de sélection de capteurs magnétiques est configurée pour sélectionner les capteurs magnétiques pour lesquels les sorties estimées parmi la pluralité de capteurs magnétiques sont supérieures à une valeur prédéterminée.

**11.** Système (50) de détection de position pour un dispositif médical (20) selon la revendication 6, dans lequel le dispositif de calcul de position est configuré pour estimer la taille et l'orientation du champ magnétique que la bobine forme au niveau des positions des capteurs magnétiques individuels sur la base de la position et de l'orientation déterminées par le calcul ; et l'unité (56) de sélection de capteurs magnétiques est configurée pour sélectionner le capteur magnétique devant être utilisé pour déterminer la position et l'orientation du dispositif médical lors du calcul suivant sur la base des sorties estimées des capteurs magnétiques individuels.

**12.** Système (50) de détection de position pour un dispositif médical (20) selon la revendication 11, dans lequel

l'unité (56) de sélection de capteurs magnétiques est configurée pour sélectionner séquentiellement un nombre prédéterminé de capteurs magnétiques pour lesquels les intensités des champs magnétiques que forme la bobine au niveau des positions de la pluralité de capteurs magnétiques sont importantes.

13. Système (50) de détection de position selon la revendication 6, dans lequel
le dispositif de calcul de position est configuré pour estimer la distance entre chaque capteur magnétique et la bobine sur la base de la position et de l'orientation du dispositif médical déterminées par le calcul ; et
l'unité (56) de sélection de capteurs magnétiques est configurée pour sélectionner le capteur magnétique devant être utilisé pour déterminer la position et l'orientation du dispositif médical lors du calcul suivant sur la base de la distance estimée entre chaque capteur magnétique et la bobine.

14. Système (50) de détection de position pour un dispositif médical (20) selon la revendication 13, dans lequel l'unité (56) de sélection de capteurs magnétiques est configurée pour sélectionner le capteur magnétique pour lequel la distance estimée entre le capteur magnétique et la bobine est plus petite qu'une valeur prédéterminée.

15. Système (50) de détection de position selon la revendication 1, dans lequel le dispositif médical (20) est un endoscope de type capsule, et l'axe longitudinal de l'endoscope de type capsule et la direction d'un axe central de la bobine sont rendus sensiblement identiques.

16. Système (10) de guidage de dispositif médical comprenant :

le système (50) de détection de position selon la revendication 1 ;
un aimant (45) installé dans le dispositif médical (20) ;
une unité (71) de génération de champ magnétique, disposée à l'extérieur de la région de fonctionnement du dispositif médical (20), qui est configurée pour générer un champ magnétique qui est réalisé pour agir sur l'aimant (45) ; et
une unité (73) de commande d'orientation de champ magnétique configurée pour commander l'orientation du champ magnétique appliqué à l'aimant (45) par l'unité (71) de génération de champ magnétique.

17. Système (10) de guidage de dispositif médical selon la revendication 16, dans lequel
l'unité (71) de génération de champ magnétique comporte trois paires d'électroaimants en forme de cadre opposées qui sont disposées dans des directions mutuellement orthogonales ;
un espace dans lequel le corps du sujet peut être positionné est prévu à l'intérieur des électroaimants ; et
la bobine d'entraînement (151) et les capteurs magnétiques (52) sont disposés dans la périphérie de l'espace dans lequel le corps du sujet peut être positionné.

18. Système (10) de guidage de dispositif médical selon la revendication 16, dans lequel
l'unité de génération de champ magnétique est configurée pour générer un champ magnétique rotatif autour du dispositif médical (20) ; et
une spirale qui convertit la force de rotation autour d'un axe longitudinal du dispositif médical (20) en force de propulsion dans la direction de l'axe longitudinal est prévue sur la surface externe du dispositif médical (20).

19. Système (10) de guidage de dispositif médical selon la revendication 18, dans lequel
le dispositif médical (20) comprend une unité d'imagerie (30) présentant un axe optique parallèle à l'axe longitudinal du dispositif médical (20) ; et
comprenant en outre une unité de commande d'image qui est configurée pour faire tourner l'image capturée par l'unité d'imagerie (30) dans une direction opposée sur la base d'une information de rotation autour de l'axe longitudinal du dispositif médical (20) par l'unité (73) de commande d'orientation de champ magnétique ; et
une unité d'affichage (82) qui est configurée pour afficher l'image capturée par l'unité d'imagerie (30) et/ou l'image tournée par l'unité de commande d'image.

20. Système (10) de guidage de dispositif médical selon la revendication 16, dans lequel
le dispositif médical (20) est un endoscope de type capsule, et l'axe longitudinal de l'endoscope de type capsule et la direction d'un axe central de la bobine sont rendus sensiblement identiques.

21. Système (50) de détection de position pour un dispositif médical (20) selon la revendication 1, comprenant en outre :

une unité de sélection de bobines d'entraînement configurée pour utiliser sélectivement une pluralité de bobines d'entraînement.

22. Système (50) de détection de position pour un dispositif médical (20) selon la revendication 1, dans lequel une pluralité de bobines d'entraînement (151) sont disposées de façon à être mutuellement orthogonales.

23. Système (10) de guidage de dispositif médical selon la revendication 16, dans lequel l'un parmi la bobine d'entraînement (151) et le capteur magnétique (52) est disposé au niveau de la partie supérieure d'un espace dans lequel le sujet peut être positionné.

FIG. 1

EP 1 755 449 B1

## FIG. 2

# FIG. 3

FIG. 4

# FIG. 5

EP 1 755 449 B1

## FIG. 6

EP 1 755 449 B1

## FIG. 7

## FIG. 8

## FIG. 9

## FIG. 10

EP 1 755 449 B1

# FIG. 11

## FIG. 12

# FIG. 13

EP 1 755 449 B1

# FIG. 14

# FIG. 15

FIG. 16

FIG. 17

FIG. 18

EP 1 755 449 B1

**EP 1 755 449 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3321235 B
- JP 2001179700 A
- JP 2002187100 A
- WO 2004014225 A
- US 5592939 A